Europäisches Patentamt
European Patent Office
Office européen des brevets

(11) Veröffentlichungsnummer: **0 009 142**
**A1**

# EUROPÄISCHE PATENTANMELDUNG

(21) Anmeldenummer: **79103150.3**

(22) Anmeldetag: **27.08.79**

(51) Int. Cl.³: **C 07 D 513/04,** A 61 K 31/54 // (C07D513/04, 333/00, 279/00)

(30) Priorität: **02.09.78 DE 2838377**

(43) Veröffentlichungstag der Anmeldung: **02.04.80 Patentblatt 80/7**

(84) Benannte Vertragsstaaten: **AT BE CH DE FR GB IT LU NL SE**

(71) Anmelder: **Dr. Karl Thomae GmbH, Postfach 720, D-7950 Biberach (Riss) (DE)**

(72) Erfinder: **Engel, Wolfhard, Dr. Dipl.-Chem., Mozartstrasse 13, D-7950 Biberach 1 (DE)**
Erfinder: **Trummlitz, Günter, Dr. Dipl.-Chem., Buchenweg 27, D-7951 Warthausen (DE)**
Erfinder: **Seeger, Ernst, Dr. Dipl.-Chem., Alpenstrasse 39, D-7950 Biberach 1 (DE)**
Erfinder: **Haarmann, Walter, Dr., Schlierholzweg 8, D-7950 Biberach 1 (DE)**
Erfinder: **Engelhardt, Günther, Dr., Unterer Bühl 18, D-7950 Biberach 1 (DE)**
Erfinder: **Zimmermann, Rainer, Dr. Dipl.-Biochem., Amriswilstrasse 7, D-7950 Biberach 1 (DE)**

(54) **Neue 4-Hydroxy-2H(1)benzothieno(2,3-e)-1,2-thiazin-3-carboxamid-1,1-dioxide sowie deren Salze, deren Verwendung und Verfahren zu ihrer Herstellung.**

(57) Es werden neue 4-Hydroxy-2H[1]benzothieno[2,3-e]-1,2-thiazin-3-carboxamid-1,1-dioxide der allgemeinen Formel

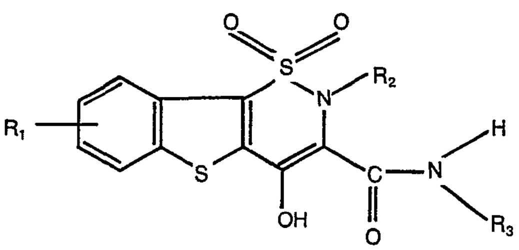
, (I)

in der $R_1$ Wasserstoff, Halogen oder niedere Alkylgruppen, $R_2$ Wasserstoff oder niedere Alkylgruppen und $R_3$ aromatische Kohlenwasserstoffreste oder heteroaromatische Reste mit weiteren Stickstoff-, Sauerstoff oder Schwefelatomen im Ring bedeuten, sowie deren Salze mit anorganischen oder organischen Basen, des weiteren ein Verfahren zur Herstellung dieser neuen Verbindungen und deren medizinische Verwendungsmöglichkeiten beschrieben. Die Verbindungen sind antiphlogistisch wirksam und üben eine Hemmwirkung auf die Blutplättchenaggregation aus. Es wird ein Weg zu ihrer Herstellung, ausgehend von entsprechend substituierten Benzo[b]thiophen-2-carbonsäurehalogeniden, aufgezeigt.

EP 0 009 142 A1

Case 5/741
Dr.Bu/ma/st

DR. KARL THOMAE GMBH, BIBERACH AN DER RISS

Neue 4-Hydroxy-2H[1]benzothieno[2,3-e]-1,2-thiazin-3-carboxamid-1,1-dioxide sowie deren Salze, deren Verwendung und Verfahren zu ihrer Herstellung

Gegenstand der vorliegenden Erfindung sind neue 4-Hydroxy-2H[1]benzothieno[2,3-e]-1,2-thiazin-3-carboxamid-1,1-dioxide der allgemeinen Formel

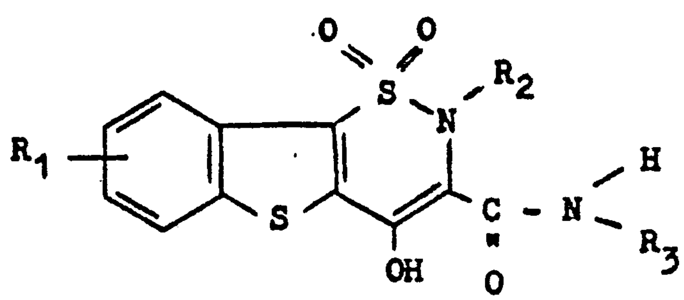

,(I)

sowie deren physiologisch verträgliche Salze mit anorganischen oder organischen Basen, deren Verwendung und Verfahren zu ihrer Herstellung. Diese Verbindungen weisen wertvolle pharmakologische Eigenschaften auf, insbesondere antiphlogistische Eigenschaften und/oder eine stark hemmende Wirkung auf die Blutplättchenaggregation.

In der obigen allgemeinen Formel I bedeuten:

$R_1$ ein Wasserstoff- oder Halogenatom oder eine Alkylgruppe mit 1 bis 3 Kohlenstoffatomen,

$R_2$ ein Wasserstoffatom oder eine Alkylgruppe mit 1 bis 3 Kohlenstoffatomen und

$R_3$ einen aromatischen Kohlenwasserstoffrest mit 6 bis 10 Kohlenstoffatomen oder einen 1 oder 2 Stickstoffatome und/oder ein Sauerstoff- oder Schwefelatom enthaltenden heteroaromatischen Rest mit 2 bis 9 Kohlenstoffatomen, wobei die oben genannten aromatischen Reste durch 1 oder 2 Alkylreste mit 1 bis 6 Kohlenstoffatomen, durch ein Halogenatom, durch eine Hydroxy-, Trifluormethyl- oder Halogenphenylgruppe oder durch eine Alkoxygruppe mit 1 bis 3 Kohlenstoffatomen substituiert sein können.

Als aromatischer Rest kommt hierbei insbesondere die Phenyl-, Naphthyl-, Pyridyl-, Pyrimidyl-, Pyrazinyl-, Pyridazinyl-, Oxazolyl-, Isoxazolyl-, Thiazolyl-, Isothiazolyl-, Benzothiazolyl-, 4,5,6,7-Tetrahydrobenzo-thiazolyl-, Cyclopenta-thiazolyl- oder 1,3,4-Thiadiazolylgruppe in Betracht.

Gegenstand der vorliegenden Erfindung sind somit insbesondere diejenigen Verbindungen der allgemeinen Formel I, in der

$R_1$ ein Wasserstoff-, Chloratom oder die Methylgruppe,

$R_2$ die Methylgruppe und

$R_3$ eine gegebenenfalls durch ein Chloratom substituierte Phenylgruppe, eine gegebenenfalls durch ein Chloratom, eine Hydroxy- oder Methylgruppe substituierte Pyridylgruppe, eine gegebenenfalls durch ein oder zwei Alkylgruppen mit 1 bis 4 Kohlenstoffatomen, ein Chloratom, eine Trifluormethyl- oder Chlorphenylgruppe substituierten Thiazolylrest, die Benzothiazolyl-, 5,6-Dimethyl-benzothiazolyl-, 4,5,6,7-Tetrahydro-benzothiazolyl-, 5,6-Dihydro-4H-cyclopentathiazolyl-, 5-Methyl-isoxazolyl-, Pyrimidinyl- oder Pyrazinylgruppe bedeuten.

Erfindungsgemäß lassen sich die neuen Verbindungen der allgemeinen Formel I wie folgt herstellen:

Umsetzung eines 4-Hydroxy-2H-[1]benzothieno[2,3-e]-1,2-thiazin-3-carbonsäure-Derivat der allgemeinen Formel

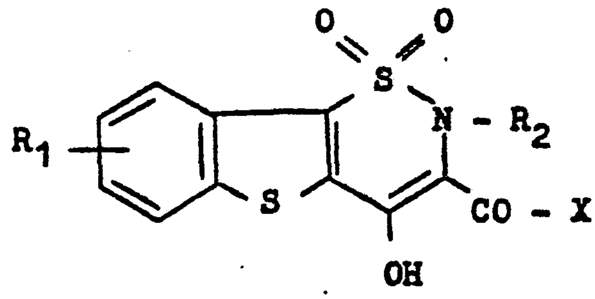

,(II)

in der

$R_1$ und $R_2$ wie eingangs definiert sind und

X eine nucleophil austauschbare Gruppe wie eine Alkoxygruppe mit 1 bis 6 Kohlenstoffatomen oder eine Aralkoxygruppe mit 7 bis 10 Kohlenstoffatomen bedeutet, mit einem Amin der allgemeinen Formel

$$NH_2 - R_3 \quad ,(III)$$

in der

$R_3$ wie eingangs definiert ist.

Die Umsetzung erfolgt zweckmäßigerweise in einem geeigneten indifferenten organischen Lösungsmittel, beispielsweise in einem aromatischen Kohlenwasserstoff wie Benzol, Toluol, Xylol, Chlorbenzol, o-Dichlorbenzol oder Tetrahydronaphthalin, in einem dipolaren aprotischen Lösungsmittel wie Dimethylformamid, Dimethylacetamid, Hexamethylphosphorsäuretriamid oder Dimethylsulfoxid, in einem Ether wie Dimethoxyethan, Diethylenglykoldimethylether oder Diphenylether, oder in einem Überschuß des eingesetzten Amins bei Temperaturen zwischen 60 und 200°C, vorzugsweise jedoch bei der Siedetemperatur des Reaktionsgemisches. Die Umsetzung kann jedoch auch ohne Lösungsmittel durchgeführt werden.

Besonders vorteilhaft wird jedoch die Umsetzung bei der Siedetemperatur des Toluols oder Xylols durchgeführt, wobei gleichzeitig der bei der Umsetzung entstehende Alkohol HX durch azeotrope Destillation oder durch Verwendung eines mit Molekularsieb beschickten Soxhlet-Extraktors entfernt wird.

Die erhaltenen Verbindungen der allgemeinen Formel I können anschließend gewünschtenfalls in ihre physiologisch verträglichen Salze mit anorganischen oder organischen Basen übergeführt werden. Als Basen kommen beispielsweise Alkalialkoholate wie Natriumäthylat, Alkalihydroxide wie Natrium- oder Kaliumhydroxid, Erdalkalihydroxide wie Calciumhydroxid, Tetraalkylammoniumhydroxide wie Tetraäthyl-ammoniumhydroxid oder Alkylamine wie Methylamin oder Cyclohexylamin in Betracht.

Die als Ausgangsstoffe verwendeten Verbindungen der allgemeinen Formel II können entsprechend dem nachfolgenden Reaktionsschema hergestellt werden:

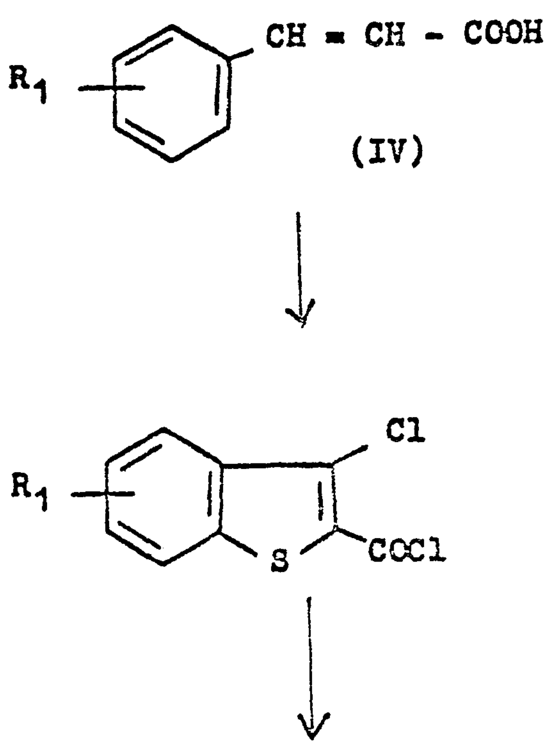

(IV)

(V)

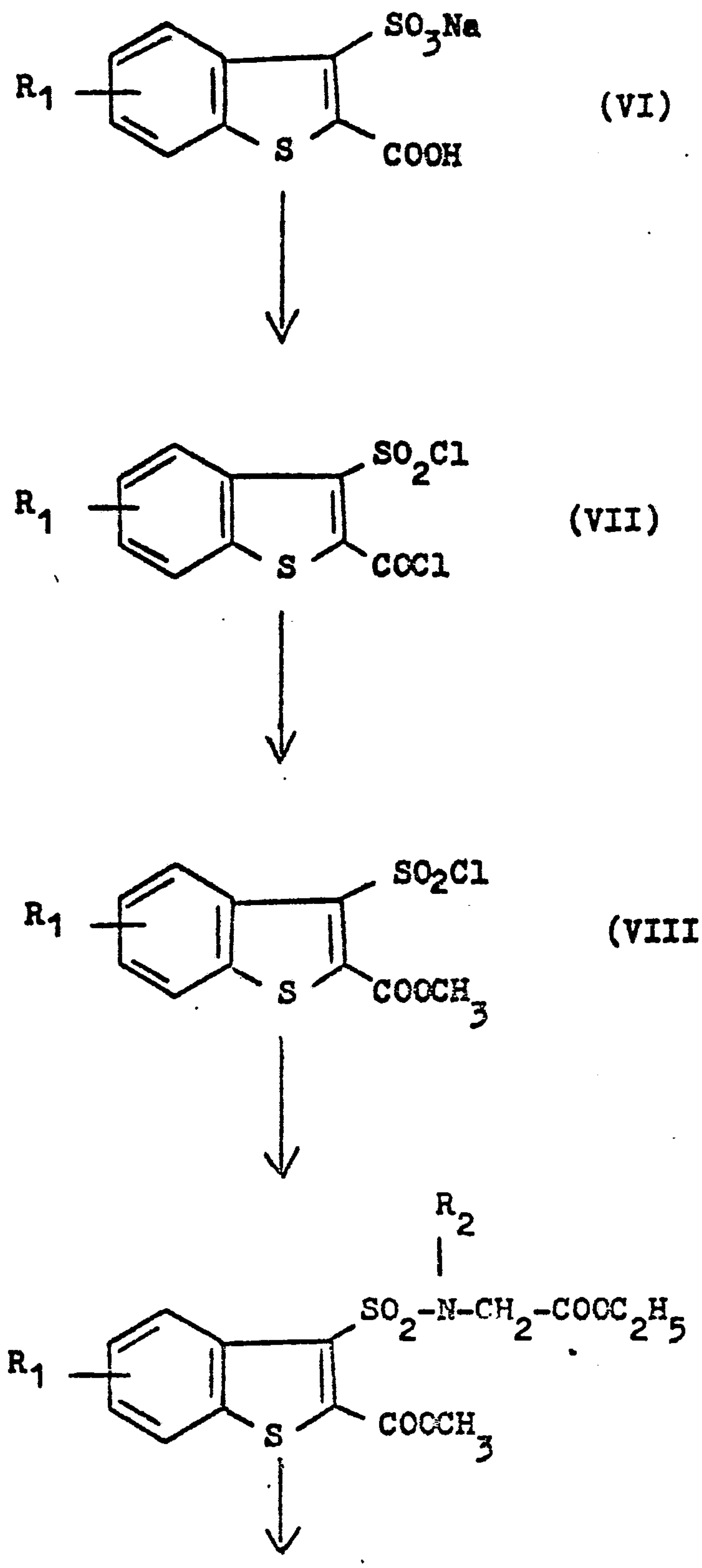

(VI)

(VII)

(VIII)

(IX)

(II)

Hierzu wird eine Zimtsäure der allgemeinen Formel IV bei erhöhten Temperaturen mit Thionylchlorid in Pyridin zu einem 3-Chlor-benzo/b/thiophen-2-carbonsäurechlorid der allgemeinen Formel V übergeführt (siehe W.B. Wright et al. in J. Het. Chem. 8, 711 (1971) und T. Higa et al. in J. org. Chem. 40, 3037 (1975) und ibid. 41, 3399 (1976)), welches anschließend alkalisch verseift wird. Das erhaltene Alkalisalz einer 3-Chlorbenzo/b/thiophen-2-carbonsäure wird in wässriger Lösung mit Natriumhydrogensulfit in Gegenwart eines Cupfer-(I)-halogenids in einem Autoklaven bei Temperaturen zwischen 135 und 150°C umgesetzt. Aus dem Reaktionsgemisch erhält man durch Ansäuern mit konz. Salzsäure und durch Zugabe von Natriumchlorid eine Verbindung der allgemeinen Formel VI, welche nach dem Trocknen mit mindestens 2 Mol Phosphorpentachlorid in Phosphoroxidtrichlorid bei Temperaturen zwischen 30°C und der Siedetemperatur des Gemisches in ein entsprechendes Säurechlorid der allgemeinen Formel VII übergeführt wird. Ein so erhaltenes Säurechlorid der allgemeinen Formel VII wird anschließend mittels Methanolyse in einen entsprechenden Ester der allgemeinen Formel VIII übergeführt, welcher durch Umsetzung mit einem entsprechenden Glycinester in eine Verbindung der allgemeinen Formel IX übergeführt wird. Die Überführung einer so erhaltenen Verbindung der allgemeinen Formel IX in eine Ausgangsverbindung der allgemeinen Formel II erfolgt durch Umsetzung mit einem Alkoholat eines Alkohols der allgemeinen Formel

$$H - X \quad ,(X)$$

in der
X wie eingangs definiert ist, in Gegenwart eines entsprechenden wasserfreien Alkohols bei Temperaturen zwischen 10 und 80°C.

Die als Ausgangsstoffe verwendeten Amine der allgemeinen Formel III sind größtenteils literaturbekannt. Beispielsweise erhält man 2-Amino-4,5,6,7-tetrahydro-benzothiazol, 2-Amino-4-(1,1-dimethylethyl)-thiazol und 2-Amino-4-(4-chlorphenyl)-thiazol entsprechend der Publikation von L. C. King et al. in J. Amer. chem. Soc. 72, 3722 (1950) durch Umsetzung von einem entsprechenden Keton, Thioharnstoff und Jod, 4-Trifluormethyl-2-thiazolamin durch Umsetzung von 1-Brom-3,3,3-trifluor-aceton mit Thioharnstoff (siehe J.B. Dickey et al. in J. org. Chem. 20, 409, 505ff (1955)) und 5-Alkyl-2-thiazolamine durch Chlorierung eines geeigneten primären Alkohols bei 0 bis 5°C und anschließende Umsetzung mit Thioharnstoff (siehe M. V. Shirsat in C. A. 54, 14230e (1960)).

Wie bereits eingangs erwähnt, besitzen die neuen Verbindungen der allgemeinen Formel I und deren physiologisch verträgliche Salze mit anorganischen oder organischen Basen wertvolle pharmakologische Eigenschaften, neben analgetischen und antipyretischen insbesondere antiphlogistische Eigenschaften und/oder eine stark hemmende Wirkung auf die Blutplättchen-Aggregation.

Beispielsweise wurden die Substanzen

A = 4-Hydroxy-2-methyl-N-(2-thiazolyl)-2H-[1]benzothieno-[2,3-e]-1,2-thiazin-3-carboxamid-1,1-dioxid,

B = 4-Hydroxy-2-methyl-N-(6-methyl-2-pyridyl)-2H-[1]benzothieno[2,3-e]-1,2-thiazin-3-carboxamid-1,1-dioxid,

C = 4-Hydroxy-2-methyl-N-(4-methyl-2-thiazolyl)-2H-[1]benzothieno[2,3-e]-1,2-thiazin-3-carboxamid-1,1-dioxid,

D = 4-Hydroxy-2-methyl-N-(4-methyl-2-pyridyl)-2H-[1]benzothieno[2,3-e]-1,2-thiazin-3-carboxamid-1,1-dioxid,

E = 2,7-Dimethyl-4-hydroxy-N-(4-methyl-2-pyridyl)-2H-[1]-benzothieno[2,3-e]-1,2-thiazin-3-carboxamid-1,1-dioxid,

F = 2,7-Dimethyl-4-hydroxy-N-(6-methyl-2-pyridyl)-2H-/1/-benzothieno/2,3-e/-1,2-thiazin-3-carboxamid-1,1-dioxid,

G = 7-Chlor-4-hydroxy-2-methyl-N-(4-methyl-2-pyridyl)-2H-/1/-benzothieno/2,3-e/-1,2-thiazin-3-carboxamid-1,1-dioxid und

H = 7-Chlor-4-hydroxy-2-methyl-N-(6-methyl-2-pyridyl)-2H-/1/benzothieno/2,3-e/-1,2-thiazin-3-carboxamid-1,1-dioxid

auf ihre antithrombotische Wirkung im Vergleich zu

V = Acetylsalicylsäure und die Substanzen A, C,

I = N-(2-Benzothiazolyl)-4-hydroxy-2-methyl-2H-/1/benzothieno/2,3-e/-1,2-thiazin-3-carboxamid-1,1-dioxid,

J = N-(5-Chlor-2-thiazolyl)-4-hydroxy-2-methyl-2H-/1/benzothieno/2,3-e/-1,2-thiazin-3-carboxamid-1,1-dioxid und

K = N-(4,5-Dimethyl-2-thiazolyl)-4-hydroxy-2-methyl-2H-/1/-benzothieno/2,3-e/-1,2-thiazin-3-carboxamid-1,1-dioxid

im Vergleich zu

W = Sudoxicam

auf ihre entzündungshemmende Wirkung wie folgt untersucht:

1. Hemmung auf die Blutplättchen-Aggregation:

Die Thrombozytenaggregation wurde nach der Methode von BORN und CROSS (J. Physiol. 170, 397, /1964/) an plättchenreichem Plasma gesunder Versuchspersonen gemessen.

Die Abnahme der optischen Dichte von Plättchensuspensionen nach Zugabe von Collagen wird photometrisch gemessen und registriert.

Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density". Die Collagen-Menge wird so gewählt, daß sich eine irreversibel verlaufende Kontrollkurve ergibt.

Die angegebenen Zahlen beziehen sich auf die "optical density" und bedeuten prozentuale Änderung der Lichtdurchlässigkeit (= % Abschwächung der Aggregation) unter Substanzeinfluß im Vergleich zur Kontrolle.

Verwendet wurde das handelsübliche Collagen der Firma Hormonchemie München.

Die folgende Tabelle enthält die nach diesem Versuch ermittelten Ergebnisse:

| Substanz | Konzentration [Mol $l^{-1}$] | Prozentuale Änderung der Lichtdurchlässigkeit d. PRP (BORN-Test) |
|---|---|---|
| A | $10^{-5}$ | 94 |
| | $10^{-6}$ | 73 |
| | $10^{-7}$ | 14 |
| B | $4.10^{-6}$ | 100 |
| | $4.10^{-7}$ | 71 |
| C | $10^{-5}$ | 93 |
| | $10^{-6}$ | 57 |

| Substanz | Konzentration [Mol $l^{-1}$] | Prozentuale Änderung der Lichtdurchlässigkeit d. PRP (BORN-Test) |
|---|---|---|
| D | $2.10^{-5}$<br>$2.10^{-6}$<br>$2.10^{-7}$ | 88<br>60<br>19 |
| E | $2.10^{-5}$<br>$2.10^{-6}$ | 72<br>34 |
| F | $2.10^{-5}$<br>$2.10^{-6}$<br>$2.10^{-7}$ | 90<br>77<br>30 |
| G | $2.10^{-5}$<br>$2.10^{-6}$ | 66<br>33 |
| H | $2.10^{-5}$<br>$2.10^{-6}$<br>$2.10^{-7}$ | 89<br>88<br>33 |
| V | $3.10^{-5}$<br>$10^{-5}$ | 45<br>13 |

2. Entzündungshemmende Wirkung:

Die entzündungshemmende Wirkung (siehe Vane in Nature New Biology 231, 232, (1971), analgetische (siehe Ferreira in Nature New Biology 240, 200 (1972)) und auch die antipyretische Wirkung (siehe Flower und Vane in Nature New Biology 240, 410 (1972)) der nichtsteroidischen Antiphlogistica kommt über den Mechanismus der Hemmung der Prostaglandinbiosynthese zustande.

Die entzündungshemmende Wirkung wurde daher als Wirkung gegenüber den Prostaglandinsynthetasen bestimmt:

Methodik:

Die Bestimmung der Prostaglandinbiosyntheserate erfolgte radiochemisch mit einer Modifikation der Methode von WHITE u. GLASSMAN (Prostaglandins 7, 123 (1974)).
Die Prostaglandinsynthetasen wurden als Mikrosomenextrakt aus Bullensamenblasen nach FLOWER et al. (Prostaglandins 4, 325 (1973)) isoliert.
Als Substrat diente $^{14}$C-Arachidonsäure. Die daraus biosynthetisierten Prostaglandine wurden von der $^{14}$C-Arachidonsäure chromatographisch abgetrennt und das Restsubstrat und Produkt radiochemisch gemessen.
Aus der nach Einwirkung der verschiedenen Konzentrationen der Prüfsubstanzen eluierten Menge von Prostaglandinen (gemessen in dpm, bezogen auf 20.000 dpm eluierte Prostaglandine + Arachidonsäure) wurde nach linearer Regressionsanalyse nach LINDER (Statistische Methoden, 4. Aufl., pp. 148-162, Birkhäuser, Basel 1964) eine $EK_{50}$ als jene Konzentration im Endansatz berechnet, die zu einer 50%igen Verminderung der Prostaglandinmenge gegenüber der in wirkstofffreien Kontrollansätzen gefundenen führte.

| Substanz | $n_1$ | $n_2$ | DMSO$^+$ % | $EK_{50}$ in Mol |
|---|---|---|---|---|
| A | 3 | 6 | 2 | 3,9 x $10^{-8}$ |
| C | 4 | 6 | 2 | 2,1 x $10^{-8}$ |
| K | 5 | 5-6 | 10 | 2,5 x $10^{-7}$ |
| I | 5 | 6 | 2 | 2,2 x $10^{-8}$ |
| J | 5 | 6 | 2 | 4,7 x $10^{-8}$ |
| W | 5 | 6 | 2 | 7,0 x $10^{-6}$ |

$n_1$ = Zahl der geprüften Konzentrationen
$n_2$ = Zahl der Proben/Konzentration
x = Konzentration des Lösungsvermittlers DMSO im Endansatz

## 3. Akute Toxizität

Die Bestimmung der akuten Toxizität erfolgte an Ratten beider Geschlechter (1:1) mit einem Gewicht von 120-150 g. Die einmalige Gabe der Prüfsubstanz erfolgte als Verreibung in 1%iger Methylzellulose (1 ml/100 g Tier) per Schlundsonde, die Nachbeobachtungszeit betrug 24 Stunden.

| Substanz | Toxizität |
|---|---|
| A | > 100 mg/kg p.o. (0 von 10 Tieren gestorben) |
| C | > 100 mg/kg p.o. (0 von 10 Tieren gestorben) |
| K | > 100 mg/kg p.o. (0 von 10 Tieren gestorben) |
| I | > 100 mg/kg p.o. (0 von 10 Tieren gestorben) |
| J | > 100 mg/kg p.o. (0 von 10 Tieren gestorben) |

Aufgrund ihrer pharmakologischen Eigenschaften eignen sich daher die neuen Verbindungen der allgemeinen Formel I und deren physiologisch verträgliche Salze mit anorganischen oder organischen Basen insbesondere zur Behandlung von entzündlichen Zuständen der verschiedensten Genese, wie posttraumatischen Entzündungs- und Schwellungszuständen, nichttraumatischen Entzündungen des Bewegungsapparates (inkl. rheumatische Entzündungen), Entzündungen und Schwellungen nach Operationen, Entzündungen der Adnexe, der Blut- und Lymphgefäße, und/oder zur Prophylaxe arterieller Thromboembolien und arterieller Verschlußkrankheiten und lassen sich hierzu zur pharmazeutischen Anwendung, gegebenenfalls in Kombination mit anderen Wirksubstanzen, in die üblichen pharmazeutischen Zubereitungen wie Dragées, Tabletten, Kapseln, Suppositorien, Pulver oder Suspensionen einarbeiten. Die Einzeldosis beträgt hierbei am Menschen 10 - 250 mg, vorzugsweise 25 - 100 mg, und die Tagesdosis 25 - 500 mg, vorzugsweise 50 - 250 mg.

Die nachfolgenden Beispiele sollen die Erfindung näher erläutern:

## Beispiel 1

**4-Hydroxy-2-methyl-N-(2-thiazolyl)-2H-/1/benzothieno/2,3-e/-1,2-thiazin-3-carboxamid-1,1-dioxid**

25,0 g (0,0768 Mol) 4-Hydroxy-2-methyl-2H-/1/benzothieno-/2,3-e/-1,2-thiazin-3-carbonsäuremethylester-1,1-dioxid und 10,0 g (0,12 Mol) 2-Thiazolamin werden in 300 $cm^3$ wasserfreiem Xylol und unter Verwendung von Stickstoff als Schutzgas 16 Stunden unter Rückfluß gekocht. Das dabei entstehende Methanol wird mit 4 Å-Molekularsieb, das sich in einem Soxhlet-Aufsatz befindet, entfernt. Man läßt erkalten und filtriert nach mehrstündigem Stehenlassen das entstandene Reaktionsprodukt ab. Nach Umkristallisation aus einem Gemisch von Dimethylformamid und 1,2-Dichlorethan erhält man 19,9 g (66 % der Theorie) an fahlgelbem 4-Hydroxy-2-methyl-N-(2-thiazolyl)-2H-/1/benzothieno/2,3-e/-1,2-thiazin-3-carboxamid-1,1-dioxid vom Schmelzpunkt 252 - 253°C (Zers.).

$C_{15}H_{11}N_3O_4S_3$ (393,47)

| | | | | | | | | |
|---|---|---|---|---|---|---|---|---|
| Ber.: | C | 45,79 | H | 2,82 | N | 10,68 | S | 24,45 |
| Gef.: | | 45,80 | | 2,85 | | 10,66 | | 24,50 |

IR(KBr): N-H 3300, C=O 1630, Amid-II 1540, $SO_2$ 1340, 1150/cm

UV(Ethanol): $\lambda_{max}$ 407 und 378 nm, Schulter bei 260 nm; nach Zusatz von KOH: $\lambda_{max}$ 285 und 414 nm.

1H-NMR ($d_6$-DMSO): $\delta$ 8,4-8,0 (2H-m, ar. H); 7,8-7,4 (2H-m, ar.H); 7,61 (1H-d, J=4,4 Hz, 4'-H); 7,26 (1H-d, J=4,4 Hz, 5'-H); 3,03 (3H-s, 2-$CH_3$) und 2 austauschbare Protonen.

Die Ausgangsverbindung erhält man über die folgenden Zwischenstufen:

a) 2-Carboxy-benzo/b/thiophen-3-sulfonsäure-natriumsalz

74,0 g (0,32 Mol) 3-Chlor-benzo/b/thiophen-2-carbonsäure-chlorid (Schmp. 112-113°C) werden mit der Lösung von 25,6 g (0,64 Mol) Natriumhydroxid in 250 $cm^3$ Wasser unter Einhaltung einer Reaktionstemperatur von 50°C 12 Stunden gerührt. Zu der dann neutral reagierenden Lösung gibt man 66,6 g (0,64 Mol) festes Natriumhydrogensulfit, stellt mit 40%iger Natronlauge gerade alkalisch und erhitzt nach Zusatz von 3,0 g (0,03 Mol) Cupfer(I)-chlorid im Autoklaven 16 Stunden auf 143°C. Nach dem Erkalten stellt man mit Salzsäure auf pH 4, filtriert und extrahiert zur Entfernung des restlichen unumgesetzten Ausgangsmaterials einmal mit 200 $cm^3$ Essigsäureethylester. Aus der wäßrigen Phase fällt nach weiterem Ansäuern auf pH 1 und Zusatz von 60 g Natriumchlorid ein farbloser Niederschlag aus, der abgenutscht, mit 30 $cm^3$ gesättigter wäßriger Natriumchlorid-Lösung nachgewaschen und scharf abgesaugt wird. Man löst in warmem Methanol, filtriert vom unlöslichen überschüssigen Natriumchlorid ab, verdampft das Lösungsmittel im Vakuum und trocknet durch Azeotropdestillation mit 50 $cm^3$ Toluol. Man erhält 43 g (48 % der Theorie) an farblosem, rohem 2-Carboxy-benzo/b/-thiophen-3-sulfonsäure-natriumsalz, das ohne weitere Reinigung weiter umgesetzt wird.

b) 2-Chlorcarbonyl-benzo/b/thiophen-3-sulfonsäurechlorid

44,0 g (0,157 Mol) des obigen 2-Carboxy-benzo/b/thiophen-3-sulfonsäure-mononatriumsalzes werden in 200 $cm^3$ Phosphoroxidchlorid suspendiert und mit 65,4 g (0,314 Mol) Phosphor-(V)-chlorid versetzt. Zur Vervollständigung der unter Chlorwasserstoff-Entwicklung verlaufenden, anfangs exothermen Reaktion erhitzt man unter Rühren noch 2 Stunden auf 100°C, läßt erkalten, filtriert von anorganischen Salzen ab und

dampft im Vakuum ein. Der Rückstand wird in 300 $cm^3$ trockenem Chloroform aufgenommen, die Lösung filtriert und abermals eingedampft. Der ölige Rückstand, bestehend aus 27 g (58 % der Theorie) rohem 2-Chlorcarbonyl-benzo/b/thiophen-3-sulfonsäurechlorid, kristallisiert beim Abkühlen aus (Schmp. 45 - 47°C) und wird ohne weitere Reinigung in die nächste Stufe eingesetzt.

c) 2-Methoxycarbonyl-benzo/b/thiophen-3-sulfonsäurechlorid

168,0 g (0,569 Mol) 2-Chlorcarbonyl-benzo/b/thiophen-3-sulfon-säurechlorid werden in 1 l wasserfreiem Chloroform gelöst, mit 27,5 g (0,858 Mol) trockenem Methanol versetzt und 3 Stunden unter Rückfluß erhitzt. Man dampft im Vakuum ein und kristallisiert den Rückstand zweimal aus wenig Methanol um. Man erhält 88,5 g (53,5 % der Theorie) an farblosem 2-Methoxycarbonyl-benzo/b/thiophen-3-sulfonsäurechlorid vom Schmelzpunkt 100-101°C.

$C_{10}H_7ClO_4S_2$ (290,75)

| | | | | | | | | |
|---|---|---|---|---|---|---|---|---|
| Ber.: | C | 41,31 | H | 2,43 | Cl | 12,19 | S | 22,06 |
| Gef.: | | 41,35 | | 2,53 | | 12,55 | | 22,90 |

IR ($CH_2Cl_2$): C=O 1740, $SO_2$ 1175, 1380/cm

UV(Ethanol): $\lambda_{max}$ 254, 305 nm; nach Zusatz von KOH: $\lambda_{max}$ 280, Schulter bei 240 nm.

1H-NMR ($CDCl_3$): $\delta$ 8,6-8,2 (1H-m, ar. H); 8,1-7,8 (1H-m, ar.H), 7,8-7,4 (2H-m, ar. H); 4,06 (3H-s, Ester-$CH_3$)

d) 3-//(Ethoxycarbonylmethyl)methylamino/sulfonyl/-benzo/b/-thiophen-2-carbonsäuremethylester

Zu der Lösung von 138,0 g (0,898 Mol) Sarcosinethylester-hydrochlorid in 300 $cm^3$ Methanol tropft man unter äußerer Kühlung mit Eiswasser 182 g (1,799 Mol) Triethylamin, dann

die Lösung von 252,0 g (0,867 Mol) 2-Methoxycarbonyl-benzo-[b]thiophen-3-sulfon-säurechlorid in einem Gemisch von 1 l Diethylether und 1 l Tetrahydrofuran. Man rührt 30 Minuten bei Zimmertemperatur, kocht 30 Minuten unter Rückfluß und engt dann die gesamte Reaktionsmischung im Vakuum ein. Den verbleibenden Rückstand nimmt man in 1 l Chloroform auf, wäscht die erhaltene Lösung nacheinander mit je 200 $cm^3$ Wasser, 5%iger wäßriger Salzsäure und abermals Wasser, trocknet sie über Natriumsulfat und dampft sie ein. Der verbleibende Rückstand wird zweimal aus Ethanol umkristallisiert. Man erhält 286 g (89 % der Theorie) an farblosem 3-[[(Ethoxycarbonylmethyl)methylamino]sulfonyl]-benzo[b]-thiophen-2-carbonsäuremethylester vom Schmelzpunkt 119 - 120°C.

$C_{15}H_{17}NO_6S_2$ (371,44)

| | | | | | | | | |
|---|---|---|---|---|---|---|---|---|
| Ber.: | C | 48,51 | H | 4,61 | N | 3,77 | S | 17,26 |
| Gef.: | | 48,45 | | 4,65 | | 3,95 | | 17,50 |

IR ($CH_2Cl_2$): $\nu$ C=O 1740, $SO_2$ 1150, 1350/cm

UV (Ethanol): $\lambda_{max}$ 290, Schulter bei 245 nm

1H-NMR ($CDCl_3$): $\delta$ 8,6-8,2 (1H-m, ar. H); 8,0-7,7 (1H-m, ar. H);77,7-7,3 (2H-m, ar. H); 4,22 (2H-s, N-$CH_2$-C); 3,99 (3H-s, $OCH_3$); 4,02 (2H-q; J = 7,2 Hz, -O-$CH_2$-C), 3,11 (3H-s; $NCH_3$); 1,10 (3H-t, J = 7,2 Hz, -O-C-$CH_3$)

e) 4-Hydroxy-2-methyl-2H-[1]benzothieno[2,3-e]-1,2-thiazin-3-carbon-säuremethylester-1,1-dioxid

Zu der Suspension von 286 g (0,77 Mol) 3-[[(Ethoxycarbonyl-methyl)methylamino]sulfonyl]-benzo[b]thiophen-2-carbonsäure-methylester in 1000 $cm^3$ wasserfreiem Methanol gibt man unter Inertgasschutz 42,0 g (0,78 Mol) festes Natriummethanolat, rührt 30 Min. bei Raumtemperatur und kocht 1 Stunde

unter Rückfluß. Das Lösungsmittel wird weitgehend abdestilliert, der Rückstand in 500 ml eiskalte 2 N Salzsäure eingerührt. Das ausgefallene Produkt wird abfiltriert, in 400 $cm^3$ Chloroform aufgenommen, die Lösung über Natriumsulfat getrocknet und im Vakuum eingeengt. Nach dem Umkristallisieren aus zweimal je 100 $cm^3$ Methanol erhält man 116 g (46 % der Theorie) an fahlgelben Kristallen vom Schmelzpunkt 177-178°C.

$C_{13}H_{11}NO_5S_2$ (325,36)

| | | | | | | | | |
|---|---|---|---|---|---|---|---|---|
| Ber.: | C | 47,99 | H | 3,41 | N | 4,30 | S | 19,71 |
| Gef.: | | 48,45 | | 3,55 | | 4,68 | | 19,85 |

IR ($CH_2Cl_2$): OH breit, assoziiert, C=O 1660, $SO_2$ 1160, 1350/cm

UV(Ethanol): $\lambda_{max}$ 345, Schulter bei 255 nm; auf Zusatz von KOH: $\lambda_{max}$ 235, 290, 385, 405 nm

1H-NMR ($CDCl_3$): $\delta$ 11,84 (1H-s, austauschbares H); 8,5-8,1 (1H-m, ar. H); 8,1-7,8 (1H-m, ar. H); 7,7-7,4 (2H-m, ar. H), 3,99 (3H-s, $OCH_3$); 3,10 (3H-s, $N-CH_3$)

## Beispiel 2

4-Hydroxy-2-methyl-N-(2-pyridyl)-2H-[1]benzothieno[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäuremethylester-1,1-dioxid und 2-Amino-pyridin in einer Ausbeute von 52 % der Theorie.

Schmelzpunkt: 224-225°C (Zersetzung) nach Umkristallisation aus Ethylenchlorid.

$C_{17}H_{13}N_3O_4S_2$ (387,44)

| | | | | | | | | |
|---|---|---|---|---|---|---|---|---|
| Ber.: | C | 52,70 | H | 3,38 | N | 10,85 | S | 16,55 |
| Gef.: | | 52,30 | | 3,41 | | 11,0 | | 16,70 |

IR($CH_2Cl_2$): OH, NH breit assoziiert, C=O 1630-1640, $SO_2$ 1150, 1340/cm

UV (Ethanol): $\lambda_{max}$ 367, 403 nm; auf Zusatz von KOH: $\lambda_{max}$ 413 nm

1H-NMR ($d_6$-DMSO): $\delta$ 14,41 (1H-s, austauschbares H); 8,5-7,0 (8H-m, ar. H); 5,0-3,8 (1H-m, breit, austauschbares H); 2,98 (3H-s, N-$CH_3$)

Beispiel 3

4-Hydroxy-2-methyl-N-(6-methyl-2-pyridyl)-2H-[1]benzothieno-[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäuremethylester-1,1-dioxid und 2-Amino-6-methyl-pyridin in einer Ausbeute von 33 % der Theorie.
Schmelzpunkt: 233-234°C (Zersetzung) nach dem Umkristallisieren aus Dimethylformamid.

$C_{18}H_{15}N_3O_4S_2$ (401,47)

| | | | | | | | | |
|---|---|---|---|---|---|---|---|---|
| Ber.: | C | 53,85 | H | 3,77 | N | 10,47 | S | 15,97 |
| Gef.: | | 53,70 | | 3,95 | | 10,25 | | 15,80 |

Beispiel 4

4-Hydroxy-2-methyl-N-(4-methyl-2-thiazolyl)-2H-[1]benzothieno-[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäuremethylester-1,1-dioxid und 2-Amino-4-methyl-thiazol in einer Ausbeute von 66 % der Theorie.
Schmelzpunkt: 243 - 244°C (Zersetzung) nach dem Umkristallisieren aus Ethylenchlorid.

$C_{16}H_{13}N_3O_4S_3$ (407,49)

| | | | | | | | | |
|---|---|---|---|---|---|---|---|---|
| Ber.: | C | 47,16 | H | 3,22 | N | 10,30 | S | 23,61 |
| Gef.: | | 47,00 | | 3,30 | | 10,37 | | 23,70 |

Beispiel 5

N-(2-Benzothiazolyl)-4-hydroxy-2-methyl-2H-[1]benzothieno-[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäureethylester-1,1-dioxid und 2-Amino-benzothiazol in einer Ausbeute von 70 % der Theorie.
Schmelzpunkt: 255 - 256°C (Zersetzung) nach dem Umkristallisieren aus Dimethylformamid.

$C_{19}H_{13}N_3O_4S_3$ (443,52)

| | | | | | | | | |
|---|---|---|---|---|---|---|---|---|
| Ber.: | C | 51,45 | H | 2,95 | N | 9,47 | S | 21,69 |
| Gef.: | | 51,50 | | 3,00 | | 9,42 | | 21,68 |

Beispiel 6

N-(4-Chlorphenyl)-4-hydroxy-2-methyl-2H-[1]benzothieno[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäurebenzylester-1,1-dioxid und 4-Chloranilin in einer Ausbeute von 52 % der Theorie.

Schmelzpunkt: 251-252°C (Zersetzung) nach dem Umkristallisieren aus Ethylenglykolmonomethylether.

$C_{18}H_{13}ClN_2O_4S_2$ (420,9)

| | | | | | | | | | | |
|---|---|---|---|---|---|---|---|---|---|---|
| Ber.: | C | 51,37 | H | 3,11 | N | 6,66 | Cl | 8,42 | S | 15,24 |
| Gef.: | | 51,40 | | 3,18 | | 6,30 | | 8,55 | | 15,55 |

Beispiel 7

4-Hydroxy-2-methyl-N-phenyl-2H-[1]benzothieno[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäuremethylester-1,1-dioxid und Anilin in einer Ausbeute von 60 % der Theorie.
Schmelzpunkt: 256 - 257°C (Zersetzung) nach dem Umkristallisieren aus Ethylenglykolmonomethylether.

$C_{18}H_{14}N_2O_4S_2$ (386,45)

| | | | | | | | | |
|---|---|---|---|---|---|---|---|---|
| Ber.: | C | 55,44 | H | 3,65 | N | 7,25 | S | 16,59 |
| Gef.: | | 55,90 | | 3,68 | | 7,34 | | 16,20 |

Beispiel 8

4-Hydroxy-2-methyl-N-(5-methyl-3-isoxazolyl)-2H-[1]benzothieno-[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäuremethylester-1,1-dioxid und 5-Methyl-3-isoxazolamin in einer Ausbeute von 29 % der Theorie.
Schmelzpunkt: 245 - 246°C (Zersetzung) nach zweimaligem Umkristallisieren aus Tetrahydrofuran.

$C_{16}H_{13}N_3O_5S_2$ (391,43)

| | | | | | | | | |
|---|---|---|---|---|---|---|---|---|
| Ber.: | C | 49,10 | H | 3,35 | N | 10,74 | S | 16,38 |
| Gef.: | | 49,20 | | 3,56 | | 10,75 | | 16,78 |

Beispiel 9

4-Hydroxy-2-methyl-N-(4-methyl-2-pyridyl)-2H-/1/benzothieno-/2,3-e/-1,2-thiazin-3-carboxamid-1,1-dioxid

Hergestellt analog Beispiel 1 aus 4-Hydroxy-2-methyl-2H-/1/-benzothieno/2,3-e/-1,2-thiazin-3-carbonsäuremethylester-1,1-dioxid und 4-Methyl-2-amino-pyridin in einer Ausbeute von 31 % der Theorie.

Schmelzpunkt: 230 - 231 °C (Zersetzung) nach dem Umkristallisieren aus 1,2-Dichlorethan.

$C_{18}H_{15}N_3O_4S_2$ (401,47)

| | | | | | | | | |
|---|---|---|---|---|---|---|---|---|
| Ber.: | C | 53,85 | H | 3,77 | N | 10,47 | S | 15,97 |
| Gef.: | | 53,50 | | 3,82 | | 10,10 | | 15,50 |

IR(KBr): OH, NH breit assoziiert, C=O 1640-1650, $SO_2$ 1150, 1330/cm

UV(Ethanol): Schulter bei ca. 260, $\lambda_{max}$ 390; auf Zusatz von KOH: $\lambda_{max}$ 266, 295, 398 nm

1H-NMR ($d_6$-DMSO): $\delta$ 14,52 (1H-s, austauschbares H); 8,3-8,0 (3H-m, ar. H); 7,8-7,4 (3H-m, ar. H); 7,3-7,1 (1H-m, ar. H); 4,2-3,2 (1H-s, breit, austauschbares H); 2,94 (3H-s; N-$CH_3$); 2,48 (3H-s, >C-$CH_3$)

## Beispiel 10

**4-Hydroxy-2-methyl-N-(5-methyl-2-thiazolyl)-2H-[1]benzothieno-[2,3-e]-1,2-thiazin-3-carboxamid-1,1-dioxid**

Hergestellt analog Beispiel 1 aus 4-Hydroxy-2-methyl-2H-[1]-benzothieno[2,3-e]-1,2-thiazin-3-carbonsäuremethylester-1,1-dioxid und 5-Methyl-2-thiazolamin in einer Ausbeute von 46 % der Theorie.
Schmelzpunkt: 247-248°C nach dem Umkristallisieren aus Dimethylformamid.

$C_{16}H_{13}N_3O_4S_3$ (407,49)

| | | | | | | | | |
|---|---|---|---|---|---|---|---|---|
| Ber.: | C | 47,16 | H | 3,27 | N | 10,30 | S | 23,61 |
| Gef.: | | 47,25 | | 3,30 | | 10,24 | | 23,50 |

## Beispiel 11

**N-(4,5-Dimethyl-2-thiazolyl)-4-hydroxy-2-methyl-2H-[1]benzothieno[2,3-e]-1,2-thiazin-3-carboxamid-1,1-dioxid**

Hergestellt analog Beispiel 1 aus 4-Hydroxy-2-methyl-2H-[1]-benzothieno[2,3-e]-1,2-thiazin-3-carbonsäuremethylester-1,1-dioxid und 4,5-Dimethyl-2-thiazolamin in einer Ausbeute von 56 % der Theorie.
Schmelzpunkt: 243 - 244°C (Zersetzung) nach dem Umkristallisieren aus 1,2-Dichlorethan.

$C_{17}H_{15}N_3O_4S_3$ (421,52)

| | | | | | | | | |
|---|---|---|---|---|---|---|---|---|
| Ber.: | C | 48,44 | H | 3,59 | N | 9,97 | S | 22,82 |
| Gef.: | | 47,70 | | 3,65 | | 9,75 | | 22,18 |

Beispiel 12

N-(5-Ethyl-2-thiazolyl)-4-hydroxy-2-methyl-2H-[1]benzothieno-[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäureethylester-1,1-dioxid und 5-Ethyl-2-thiazolamin in einer Ausbeute von 38 % der Theorie.
Schmelzpunkt: 219-220°C nach dem Umkristallisieren aus 1,2-Dichlorethan.

$C_{17}H_{15}N_3O_4S_3$ (421,52)

| | | | | | | | | |
|---|---|---|---|---|---|---|---|---|
| Ber.: | C | 48,44 | H | 3,59 | N | 9,97 | S | 22,82 |
| Gef.: | | 48,20 | | 3,75 | | 9,90 | | 22,60 |

Beispiel 13

4-Hydroxy-2-methyl-N-(5-propyl-2-thiazolyl)-2H-[1]benzothieno-[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäuremethylester-1,1-dioxid und 5-Propyl-2-thiazolamin in einer Ausbeute von 37 % der Theorie.
Schmelzpunkt: 217 - 218°C (Zersetzung) nach dem Umkristallisieren aus 1,2-Dichlorethan.

$C_{18}H_{17}N_3O_4S_3$ (435,55)

| | | | | | | | | |
|---|---|---|---|---|---|---|---|---|
| Ber.: | C | 49,64 | H | 3,93 | N | 9,65 | S | 22,09 |
| Gef.: | | 49,70 | | 4,02 | | 9,61 | | 22,02 |

Beispiel 14

N-[4-(1,1-Dimethylethyl)-2-thiazolyl]-4-hydroxy-2-methyl-2H-[1]benzothieno-[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäuremethylester-1,1-dioxid und 4-(1,1-Dimethylethyl)-2-thiazolamin in einer Ausbeute von 33 % der Theorie.
Schmelzpunkt: 214 - 215°C (Zersetzung) nach dem Umkristallisieren aus 1,2-Dichlorethan.

$C_{19}H_{19}N_3O_4S_3$ (449,57)

| | | | | | | | | |
|---|---|---|---|---|---|---|---|---|
| Ber.: | C | 50,76 | H | 4,26 | N | 9,35 | S | 21,40 |
| Gef.: | | 51,10 | | 4,38 | | 9,26 | | 21,60 |

Beispiel 15

N-(5-Chlor-2-thiazolyl)-4-hydroxy-2-methyl-2H-[1]benzothieno-[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäuremethylester-1,1-dioxid und 5-Chlor-2-thiazolamin in einer Ausbeute von 34 % der Theorie.
Schmelzpunkt: 252-253°C (Zersetzung) nach dem Umkristallisieren aus einem Gemisch von Dimethylformamid und Methanol.

$C_{15}H_{10}ClN_3O_4S_3$ (427,91)

| | | | | | | | | | | |
|---|---|---|---|---|---|---|---|---|---|---|
| Ber.: | C | 42,10 | H | 2,36 | N | 9,82 | Cl | 8,29 | S | 22,48 |
| Gef.: | | 42,15 | | 2,45 | | 9,70 | | 8,63 | | 22,40 |

Beispiel 16

N-/4-(4-Chlorphenyl)-2-thiazolyl7-4-hydroxy-2-methyl-2H-/17-benzothieno/2,3-e7-1,2-thiazin-3-carboxamid-1,1-dioxid

Hergestellt analog Beispiel 1 aus 4-Hydroxy-2-methyl-2H-/17-benzothieno/2,3-e7-1,2-thiazin-3-carbonsäureethylester-1,1-dioxid und 4-(4-Chlorphenyl)-2-thiazolamin in einer Ausbeute von 40 % der Theorie.
Schmelzpunkt: 260-261°C (Zersetzung) nach dem Umkristallisieren aus 1,2-Dichlorethan.

$C_{21}H_{14}ClN_3O_4S_3$ (504,0)

| | | | | | | | | | | |
|---|---|---|---|---|---|---|---|---|---|---|
| Ber.: | C | 50,05 | H | 2,80 | N | 8,34 | Cl | 7,03 | S | 19,09 |
| Gef.: | | 50,00 | | 2,95 | | 8,21 | | 7,21 | | 18,72 |

Beispiel 17

4-Hydroxy-2-methyl-N-(4,5,6,7-tetrahydro-2-benzothiazolyl)-2H-/17benzothieno/2,3-e7-1,2-thiazin-3-carboxamid-1,1-dioxid

Hergestellt analog Beispiel 1 aus 4-Hydroxy-2-methyl-2H-/17-benzothieno/2,3-e7-1,2-thiazin-3-carbonsäurepropylester-1,1-dioxid und 2-Amino-4,5,6,7-tetrahydro-benzothiazol in einer Ausbeute von 58 % der Theorie.
Schmelzpunkt: 241-242°C (Zersetzung) nach dem Umkristallisieren aus Dimethylformamid.

$C_{19}H_{17}N_3O_4S_3$ (447,56)

| | | | | | | | | |
|---|---|---|---|---|---|---|---|---|
| Ber.: | C | 50,99 | H | 3,83 | N | 9,39 | S | 21,49 |
| Gef.: | | 51,00 | | 4,08 | | 9,39 | | 21,35 |

Beispiel 18

N-(5,6-Dihydro-4H-cyclopentathiazol-2-yl)-4-hydroxy-2-methyl-2H-/1/benzothieno/2,3-e/-1,2-thiazin-3-carboxamid-1,1-dioxid

Hergestellt analog Beispiel 1 aus 4-Hydroxy-2-methyl-2H-/1/-benzothieno/2,3-e/-1,2-thiazin-3-carbonsäuremethylester-1,1-dioxid und 5,6-Dihydro-4H-cyclopentathiazol-2-amin in einer Ausbeute von 57 % der Theorie.
Schmelzpunkt: 258 - 259°C (Zersetzung) nach dem Umkristallisieren aus Dimethylformamid.

$C_{18}H_{15}N_3O_4S_3$ (433,53)

| | | | | | | | | |
|---|---|---|---|---|---|---|---|---|
| Ber.: | C | 49,87 | H | 3,49 | N | 9,69 | S | 22,19 |
| Gef.: | | 49,30 | | 3,51 | | 9,69 | | 21,85 |

Beispiel 19

N-(5,6-Dimethyl-2-benzothiazolyl)-4-hydroxy-2-methyl-2H-/1/-benzothieno/2,3-e/-1,2-thiazin-3-carboxamid-1,1-dioxid

Hergestellt analog Beispiel 1 aus 4-Hydroxy-2-methyl-2H-/1/-benzothieno/2,3-e/-1,2-thiazin-3-carbonsäurebenzylester-1,1-dioxid und 5,6-Dimethyl-2-benzothiazolamin in einer Ausbeute von 80 % der Theorie.
Schmelzpunkt: 267-268°C (Zersetzung) nach dem Umkristallisieren aus Dimethylformamid.

$C_{21}H_{17}N_3O_4S_3$ (471,58)

| | | | | | | | | |
|---|---|---|---|---|---|---|---|---|
| Ber.: | C | 53,49 | H | 3,63 | N | 8,91 | S | 20,40 |
| Gef.: | | 53,20 | | 3,66 | | 9,18 | | 20,30 |

Beispiel 20

4-Hydroxy-2-methyl-N-(5-methyl-1,3,4-thiadiazol-2-yl)-2H-/1/-benzothieno/2,3-e/-1,2-thiazin-3-carboxamid-1,1-dioxid

Hergestellt analog Beispiel 1 aus 4-Hydroxy-2-methyl-2H-/1/-benzothieno/2,3-e/-1,2-thiazin-3-carbonsäuremethylester-1,1-dioxid und 2-Amino-5-methyl-1,3,4-thiadiazol in einer Ausbeute von 65 % der Theorie.
Schmelzpunkt: 253-254°C (Zersetzung) nach dem Umkristallisieren aus einem Gemisch von Dimethylformamid und Methanol.

$C_{15}H_{12}N_4O_4S_3$ (408,48)

| | | | | | | | | |
|---|---|---|---|---|---|---|---|---|
| Ber.: | C | 44,11 | H | 2,96 | N | 13,72 | S | 23,55 |
| Gef.: | | 44,30 | | 3,12 | | 13,50 | | 23,25 |

Beispiel 21

N-(5-Chlor-2-pyridyl)-4-hydroxy-2-methyl-2H-/1/benzothieno-/2,3-e/-1,2-thiazin-3-carboxamid-1,1-dioxid

Hergestellt analog Beispiel 1 aus 4-Hydroxy-2-methyl-2H-/1/-benzothieno/2,3-e/-1,2-thiazin-3-carbonsäuremethylester-1,1-dioxid und 2-Amino-5-chlor-pyridin in einer Ausbeute von 60 % der Theorie.
Schmelzpunkt: 232-233°C (Zersetzung) nach dem Umkristallisieren aus Dimethylformamid.

$C_{17}H_{12}ClN_3O_4S_2$ (421,89)

| | | | | | | | | | | |
|---|---|---|---|---|---|---|---|---|---|---|
| Ber.: | C | 48,40 | H | 2,87 | N | 9,96 | Cl | 8,40 | S | 15,20 |
| Gef.: | | 48,30 | | 2,87 | | 9,88 | | 8,73 | | 15,02 |

Beispiel 22

4-Hydroxy-2-methyl-N-(2-pyrimidinyl)-2H-/1/benzothieno/2,3-e/-1,2-thiazin-3-carboxamid-1,1-dioxid

Hergestellt analog Beispiel 1 aus 4-Hydroxy-2-methyl-2H-/1/-benzothieno/2,3-e/-1,2-thiazin-3-carbonsäuremethylester-1,1-dioxid und 2-Amino-pyrimidin in einer Ausbeute von 39 % der Theorie.
Schmelzpunkt: 225-226°C (Zersetzung) nach dem Umkristallisieren aus Dimethylformamid.

$C_{16}H_{12}N_4O_4S_2$ (388,43)

| | | | | | | | | |
|---|---|---|---|---|---|---|---|---|
| Ber.: | C | 49,48 | H | 3,11 | N | 14,42 | S | 16,51 |
| Gef.: | | 49,20 | | 3,29 | | 14,30 | | 16,20 |

Beispiel 23

4-Hydroxy-2-methyl-N-(pyrazinyl)-2H-/1/benzothieno/2,3-e/-1,2-thiazin-3-carboxamid-1,1-dioxid

Hergestellt analog Beispiel 1 aus 4-Hydroxy-2-methyl-2H-/1/-benzothieno/2,3-e/-1,2-thiazin-3-carbonsäureethylester-1,1-dioxid und Amino-pyrazin in einer Ausbeute von 52 % der Theorie.
Schmelzpunkt: 234-235°C (Zersetzung) nach dem Umkristallisieren aus einem Gemisch von Dimethylformamid und Methanol.

$C_{16}H_{12}N_4O_4S_2$ (388,43)

| | | | | | | | | |
|---|---|---|---|---|---|---|---|---|
| Ber.: | C | 49,48 | H | 3,11 | N | 14,42 | S | 16,51 |
| Gef.: | | 49,60 | | 3,14 | | 14,27 | | 16,30 |

Beispiel 24

4-Hydroxy-2-methyl-N-(4-trifluormethyl-2-thiazolyl)-2H-[1]benzothieno[2,3-e]-1,2-thiazin-3-carboxamid-1,1-dioxid

Hergestellt analog Beispiel 1 aus 4-Hydroxy-2-methyl-2H-[1]-benzothieno[2,3-e]-1,2-thiazin-3-carbonsäuremethylester-1,1-dioxid und 4-Trifluormethyl-2-thiazolamin in einer Ausbeute von 53 % der Theorie.
Schmelzpunkt: 225-226°C nach dem Umkristallisieren aus 1,2-Dichlorethan.

$C_{16}H_{10}F_3N_3O_4S_3$ (461,47)

| | | | | | | | | |
|---|---|---|---|---|---|---|---|---|
| Ber.: | C | 41,65 | H | 2,18 | N | 9,11 | S | 20,84 |
| Gef.: | | 41,70 | | 2,41 | | 8,73 | | 21,25 |

Beispiel 25

2,7-Dimethyl-4-hydroxy-N-(2-pyridyl)-2H-[1]benzothieno[2,3-e]-1,2-thiazin-3-carboxamid-1,1-dioxid

Hergestellt analog Beispiel 1 aus 2,7-Dimethyl-4-hydroxy-2H-[1]benzothieno[2,3-e]-1,2-thiazin-3-carbonsäuremethylester-1,1-dioxid und 2-Amino-pyridin in einer Ausbeute von 53 % der Theorie.
Schmelzpunkt: 219-220°C (Zersetzung) nach dem Umkristallisieren aus 1,2-Dichlorethan.

$C_{18}H_{15}N_3O_4S_2$ (401,47)

| | | | | | | | | |
|---|---|---|---|---|---|---|---|---|
| Ber.: | C | 53,85 | H | 3,77 | N | 10,47 | S | 15,97 |
| Gef.: | | 53,70 | | 3,86 | | 10,40 | | 15,80 |

IR (KBr): OH, NH breit, assoziiert, C=O 1640, $SO_2$ 1140, 1330/cm

UV(Ethanol): Schulter bei 260, $\lambda_{max}$ 370 nm; auf Zusatz von KOH: $\lambda_{max}$ 265, 299, 414 nm

1H-NMR ($d_6$-DMSO): $\delta$ 8,5-7,1 (7H-m, ar. H); 5,5-4,0 (1H-s, breit, austauschbares H); 3,00 (3H-s, $N-CH_3$); 2,52 (3H-s, $>C-CH_3$)

Die Ausgangsverbindung erhält man über die folgenden Zwischenstufen:

a) 2-Carboxy-6-methyl-benzo/b/thiophen-3-sulfonsäure-mononatriumsalz

Hergestellt analog Beispiel 1a) aus 3-Chlor-6-methyl-benzo/b/thiophen-2-carbonsäurechlorid (Schmelzpunkt: 124,5-126°C) durch Verseifung mit wäßriger Natriumhydroxid-Lösung und anschließende Umsetzung mit Natriumhydrogensulfit in Gegenwart von Cupfer(I)-chlorid als Katalysator.
Ausbeute: 67 % der Theorie.

b) 2-Chlorcarbonyl-6-methyl-benzo/b/thiophen-3-sulfonsäurechlorid

Hergestellt analog Beispiel 1b) aus 2-Carboxy-6-methylbenzo/b/thiophen-3-sulfonsäure-mononatriumsalz und Phosphor-(V)-chlorid in Gegenwart von Phosphoroxidtrichlorid in einer Ausbeute von 82 % der Theorie. Das beim Erkalten kristallisierende Produkt wurde ohne weitere Reinigung in die nächste Stufe eingesetzt.

c) 2-Methoxycarbonyl-6-methyl-benzo/b/thiophen-3-sulfonsäurechlorid

Hergestellt analog Beispiel 1c) aus 2-Chlorcarbonyl-6-methyl-benzo/b/thiophen-3-sulfonsäurechlorid und Methanol in Gegenwart von Chloroform.
Ausbeute: 40 % der Theorie,
Schmelzpunkt: 115-117°C nach dem Umkristallisieren aus einem Gemisch von Methanol und Ethylacetat.

$C_{11}H_9ClO_4S_2$ (304,78)

| | | | | | | | | |
|---|---|---|---|---|---|---|---|---|
| Ber.: | C | 43,35 | H | 2,98 | Cl | 11,63 | S | 21,04 |
| Gef.: | | 43,50 | | 3,04 | | 11,60 | | 21,02 |

IR($CH_2Cl_2$): C=O 1740/cm

UV(Ethanol): $\lambda_{max}$ 260, 310 nm;
nach Zusatz von KOH: $\lambda_{max}$ 250, 295 nm.

d) 3-/(/(Ethoxycarbonylmethyl)methylamino/sulfonyl/-6-methyl-benzo/b/thiophen-2-carbonsäuremethylester

Hergestellt analog Beispiel 1d) aus 2-Methoxycarbonyl-6-methyl-benzo/b/thiophen-3-sulfonsäurechlorid, Sarcosinethylesterhydrochlorid und Triethylamin in einer Ausbeute von 80 % der Theorie.
Schmelzpunkt: 86-87°C nach dem Umkristallisieren aus Ethanol.

e) 2,7-Dimethyl-4-hydroxy-2H-/1/benzothieno/2,3-e/-1,2-thiazin-3-carbonsäuremethylester-1,1-dioxid

Hergestellt analog Beispiel 1e) aus 3-/(/(Ethoxycarbonylmethyl)-methylamino/sulfonyl/-6-methyl-benzo/b/thiophen-2-carbonsäure-methylester durch Umsetzung mit Natriummethanolat in wasserfreiem Methanol in einer Ausbeute von 51 % der Theorie.
Schmelzpunkt: 212-213°C nach dem Umkristallisieren aus Methanol.

$C_{14}H_{13}NO_5S_2$ (339,39)

| | | | | | | | | |
|---|---|---|---|---|---|---|---|---|
| Ber.: | C | 49,55 | H | 3,86 | N | 4,13 | S | 18,90 |
| Gef.: | | 49,70 | | 4,01 | | 4,14 | | 18,50 |

IR(KBr): OH 3370; C=O 1720, 1680; $SO_2$ 1155, 1340/cm

UV(Ethanol): $\lambda_{max}$ 265, 352 nm;
auf Zusatz von KOH: $\lambda_{max}$ 230, 295, 385, 408 nm

1H-NMR($CDCl_3/CD_3OD$): δ 8,22 (1H-d, J=8,4 Hz, ar H in 9-Stellung; 7,84 (1H-s, ar. H in 6-Stellung); 7,46 (1H-d, J=8,4 Hz, ar. H in 8-Stellung); 4,41 (1H-s, austauschbares H); 4,02 (3H-s, $OCH_3$); 3,11 (3H-s; $N-CH_3$); 2,54 (3H-s, $\backslash C-CH_3$)

Beispiel 26

2,7-Dimethyl-4-hydroxy-N-(2-thiazolyl)-2H-/1/benzothieno-/2,3-e/-1,2-thiazin-3-carboxamid-1,1-dioxid

Hergestellt analog Beispiel 1 aus 2,7-Dimethyl-4-hydroxy-2H-/1/-benzothieno/2,3-e/-1,2-thiazin-3-carbonsäuremethylester-1,1-dioxid und 2-Amino-thiazol in einer Ausbeute von 58 % der Theorie.

Schmelzpunkt: 248-249°C (Zersetzung) nach dem Umkristallisieren aus Dimethylformamid.

$C_{16}H_{13}N_3O_4S_3$ (407,49)

| | | | | | | | | |
|---|---|---|---|---|---|---|---|---|
| Ber.: | C | 47,16 | H | 3,22 | N | 10,31 | S | 23,61 |
| Gef.: | | 47,45 | | 3,37 | | 10,21 | | 23,20 |

Beispiel 27

2,7-Dimethyl-4-hydroxy-N-(4-methyl-2-thiazolyl)-2H-/1/benzothieno/2,3-e/-1,2-thiazin-3-carboxamid-1,1-dioxid

Hergestellt analog Beispiel 1 aus 2,7-Dimethyl-4-hydroxy-2H-/1/benzothieno/2,3-e/-1,2-thiazin-3-carbonsäureethylester-1,1-dioxid und 2-Amino-4-methyl-thiazol in einer Ausbeute von 40 % der Theorie.

Schmelzpunkt: 245-246°C (Zersetzung) nach dem Umkristallisieren aus 1,2-Dichlorethan.

$C_{17}H_{15}N_3O_4S_3$ (421,52)

| | | | | | | | | |
|---|---|---|---|---|---|---|---|---|
| Ber.: | C | 48,44 | H | 3,59 | N | 9,97 | S | 22,82 |
| Gef.: | | 48,50 | | 3,64 | | 10,07 | | 23,30 |

Beispiel 28

2,7-Dimethyl-4-hydroxy-N-(5-methyl-2-thiazolyl)-2H-[1]benzothieno[2,3-e]-1,2-thiazin-3-carboxamid-1,1-dioxid

Hergestellt analog Beispiel 1 aus 2,7-Dimethyl-4-hydroxy-2H-[1]benzothieno[2,3-e]-1,2-thiazin-3-carbonsäuremethylester-1,1-dioxid und 2-Amino-5-methyl-thiazol in einer Ausbeute von 64 % der Theorie.

Schmelzpunkt: 251 - 252°C (Zersetzung) nach dem Umkristallisieren aus Dimethylformamid.

$C_{17}H_{15}N_3O_4S_3$ (421,52)

| | | | | | | | | |
|---|---|---|---|---|---|---|---|---|
| Ber.: | C | 48,44 | H | 3,59 | N | 9,97 | S | 22,82 |
| Gef.: | | 48,80 | | 3,80 | | 9,93 | | 22,70 |

Beispiel 29

2,7-Dimethyl-4-hydroxy-N-(pyrazinyl)-2H-[1]benzothieno[2,3-e]-1,2-thiazin-3-carboxamid-1,1-dioxid

Hergestellt analog Beispiel 1 aus 2,7-Dimethyl-4-hydroxy-2H-[1]benzothieno[2,3-e]-1,2-thiazin-3-carbonsäuremethylester-1,1-dioxid und Amino-pyrazin in einer Ausbeute von 39 % der Theorie.

Schmelzpunkt: 243-244°C (Zersetzung) nach dem Umkristallisieren aus Dimethylformamid.

$C_{17}H_{14}N_4O_4S_2$ (402,46)

| | | | | | | | | |
|---|---|---|---|---|---|---|---|---|
| Ber.: | C | 50,74 | H | 3,51 | N | 13,92 | S | 15,93 |
| Gef.: | | 50,40 | | 3,57 | | 13,62 | | 16,60 |

Beispiel 30

2,7-Dimethyl-4-hydroxy-N-(3-hydroxy-2-pyridyl)-2H-/1/benzothieno/2,3-e/-1,2-thiazin-3-carboxamid-1,1-dioxid

Hergestellt analog Beispiel 1 aus 2,7-Dimethyl-4-hydroxy-2H-/1/benzothieno/2,3-e/-1,2-thiazin-3-carbonsäuremethylester-1,1-dioxid und 2-Amino-3-hydroxy-pyridin in einer Ausbeute von 38 % der Theorie.
Schmelzpunkt: 287 - 288°C (Zersetzung) nach dem Auskochen mit Dimethylformamid.

$C_{18}H_{15}N_3O_5S_2$ (417,47)

| | | | | | | | | |
|---|---|---|---|---|---|---|---|---|
| Ber.: | C | 51,79 | H | 3,62 | N | 10,02 | S | 15,39 |
| Gef.: | | 51,50 | | 3,68 | | 10,27 | | 14,90 |

Beispiel 31

2,7-Dimethyl-4-hydroxy-N-(4-methyl-2-pyridyl)-2H-/1/benzothieno/2,3-e/-1,2-thiazin-3-carboxamid-1,1-dioxid

Hergestellt analog Beispiel 1 aus 2,7-Dimethyl-4-hydroxy-2H-/1/benzothieno/2,3-e/-1,2-thiazin-3-carbonsäuremethylester-1,1-dioxid und 2-Amino-4-methyl-pyridin in einer Ausbeute von 36 % der Theorie.
Schmelzpunkt: 240 - 241°C (Zersetzung) nach dem Umkristallisieren aus 1,2-Dichlorethan.

$C_{19}H_{17}N_3O_4S_2$ (415,49)

| | | | | | | | | |
|---|---|---|---|---|---|---|---|---|
| Ber.: | C | 54,93 | H | 4,12 | N | 10,11 | S | 15,43 |
| Gef.: | | 54,80 | | 4,31 | | 10,05 | | 15,80 |

IR(KBr): OH, NH breit, assoziiert, C=O 1650, $SO_2$ 1150, 1340/cm

UV(Ethanol): $\lambda_{max}$ 268, 380-390 nm; auf Zusatz von KOH: $\lambda_{max}$ 230, 267, 298, 390-400 nm

1H-NMR ($CDCl_3$ + 5 % $CF_3CO_2D$): $\delta$ 11,25 (2H-s, austauschbares H); 8,40 (1H-d, J=6,6 Hz, ar. H); 8,27 (1H-d, J=8,6 Hz, ar.H); 7,92 (1H-s, ar. H); 7,75 (1H-s, ar. H); 7,7-7,4 (2H-m; ar.H); 3,15 (3H-s, N-$CH_3$); 2,69 (3H-s, $\geq$C-$CH_3$); 2,63 (3H-s, $\geq$C-$CH_3$)

Beispiel 32

2,7-Dimethyl-4-hydroxy-N-(6-methyl-2-pyridyl)-2H-[1]benzothieno[2,3-e]-1,2-thiazin-3-carboxamid-1,1-dioxid

Hergestellt analog Beispiel 1 aus 2,7-Dimethyl-4-hydroxy-2H-[1]benzothieno[2,3-e]-1,2-thiazin-3-carbonsäuremethylester-1,1-dioxid und 2-Amino-6-methyl-pyridin in einer Ausbeute von 53 % der Theorie.

Schmelzpunkt: 235-236°C (Zersetzung) nach dem Umkristallisieren aus 1,2-Dichlorethan.

$C_{19}H_{17}N_3O_4S_2$ (415,49)

| | | | | | | | | |
|---|---|---|---|---|---|---|---|---|
| Ber.: | C | 54,93 | H | 4,12 | N | 10,11 | S | 15,43 |
| Gef.: | | 55,10 | | 4,14 | | 10,30 | | 15,97 |

Beispiel 33

7-Chlor-4-hydroxy-2-methyl-N-(2-pyridyl)-2H-[1]benzothieno-[2,3-e]-1,2-thiazin-3-carboxamid-1,1-dioxid

Hergestellt analog Beispiel 1 aus 7-Chlor-4-hydroxy-2-methyl-2H-[1]benzothieno[2,3-e]-1,2-thiazin-3-carbonsäuremethylester-1,1-dioxid und 2-Amino-pyridin in einer Ausbeute von 40 % der Theorie.

Schmelzpunkt: 231-232°C (Zersetzung) nach dem Umkristallisieren aus 1,2-Dichlorethan.

$C_{17}H_{12}ClN_3O_4S_2$ (421,89)

| | | | | | | | | | | |
|---|---|---|---|---|---|---|---|---|---|---|
| Ber.: | C | 48,40 | H | 2,87 | N | 9,96 | S | 15,20 | Cl | 8,40 |
| Gef.: | | 48,40 | | 3,34 | | 9,94 | | 14,45 | | 8,55 |

IR (KBr): OH, NH breit, assoziiert, C=O 1640, $SO_2$ 1140, 1330/cm

UV (Ethanol): $\lambda_{max}$ 375 nm ($\varepsilon$ = 0,17); auf Zusatz von KOH: $\lambda_{max}$ 298 ($\varepsilon$ = 0,37), 400 nm ($\varepsilon$ = 0,36)

Die Ausgangsverbindung erhält man über die folgenden Zwischenstufen:

a) 2-Carboxy-6-chlor-benzo[b]thiophen-3-sulfonsäure-mononatriumsalz

Hergestellt analog Beispiel 1a) aus 3,6-Dichlor-benzo[b]-thiophen-2-carbonsäurechlorid (Schmelzpunkt: 132-133°C) durch Verseifung mit wäßriger Natriumhydroxid-Lösung und anschließende Umsetzung mit Natriumhydrogensulfit in Gegenwart von Cupfer(I)-chlorid als Katalysator.
Ausbeute: 68 % der Theorie.

b) <u>6-Chlor-2-chlorcarbonyl-benzo[b]thiophen-3-sulfonsäurechlorid</u>

Hergestellt analog Beispiel 1b) aus 2-Carboxy-6-chlor-benzo[b]thiophen-3-sulfonsäure-mononatriumsalz und Phosphor(V)-chlorid in Gegenwart von Phosphoroxidtrichlorid in einer Ausbeute von 72 % der Theorie.
Das beim Erkalten kristallisierende Produkt wurde ohne weitere Reinigung in die nächste Stufe eingesetzt.

c) <u>6-Chlor-2-methoxycarbonyl-benzo[b]thiophen-3-sulfonsäurechlorid</u>

Hergestellt analog Beispiel 1c) aus 6-Chlor-2-chlorcarbonyl-benzo[b]thiophen-3-sulfonsäurechlorid und Methanol in Gegenwart von Chloroform.
Ausbeute: 69 % der Theorie,
Schmelzpunkt: 99-100°C nach dem Umkristallisieren aus Methanol.

$C_{10}H_6Cl_2O_4S_2$ (325,19)

| | | | | |
|---|---|---|---|---|
| Ber.: | C 36,94 | H 1,86 | Cl 21,80 | S 19,72 |
| Gef.: | 37,15 | 1,95 | 21,70 | 19,68 |

IR ($CH_2Cl_2$): C=O 1740, $SO_2$ 1170, 1375/cm
UV (Ethanol): $\lambda_{max}$ 256 ($\varepsilon$ = 0,37), 309 nm ($\varepsilon$ = 0,29);
nach Zusatz von KOH: $\lambda_{max}$ 247 ($\varepsilon$ = 0,35), 290 nm ($\varepsilon$ = 0,27)

d) <u>6-Chlor-3-[[(ethoxycarbonylmethyl)methylamino]sulfonyl]-benzo[b]thiophen-2-carbonsäuremethylester</u>

Hergestellt analog Beispiel 1d) aus 6-Chlor-2-methoxycarbonyl-benzo[b]thiophen-3-sulfonsäurechlorid, Sarcosinmethylester-hydrochlorid und Triethylamin in einer Ausbeute von 61 % der Theorie.
Schmelzpunkt: 77-78°C nach dem Umkristallisieren aus Methanol.

e) 7-Chlor-4-hydroxy-2-methyl-2H-/1/benzothieno/2,3-e/-1,2-thiazin-3-carbonsäuremethylester-1,1-dioxid

Hergestellt analog Beispiel 1e) aus 6-Chlor-3-//(ethoxycarbonyl-methyl)methylamino/sulfonyl/-benzo/b/thiophen-2-carbonsäure-methylester durch Umsetzung mit Natriummethanolat in wasserfreiem Methanol in einer Ausbeute von 35 % der Theorie.
Schmelzpunkt: 238 - 239°C (Zersetzung) nach dem Umkristallisieren aus Methanol.

$C_{13}H_{10}ClNO_5S_2$ (359,8)

| | | | | | | | | | | |
|---|---|---|---|---|---|---|---|---|---|---|
| Ber.: | C | 43,40 | H | 2,80 | Cl | 9,85 | N | 3,89 | S | 17,82 |
| Gef.: | | 43,30 | | 2,69 | | 10,32 | | 3,89 | | 18,57 |

IR ($CH_2Cl_2$): OH, breit, assoziiert; C=O 1660, $SO_2$ 1150, 1350/cm

UV (Ethanol): $\lambda_{max}$ 267 ($\varepsilon$ = 0,15), 347 nm ($\varepsilon$ = 0,60); auf Zusatz von KOH: $\lambda_{max}$.233 ($\varepsilon$ = 0,63), 296 ($\varepsilon$ = 0,30) und 382 nm ($\varepsilon$ = 0,30)

1H-NMR ($d_6$-DMSO): $\delta$ 8,65 (1H-d, J = 2 Hz, ar. H in 6-Stellung); 8,27 (1H-d, J = 9 Hz, ar. H in 9-Stellung); 7,84 (1H-d von d, J = 2 und 9 Hz, ar. H in 8-Stellung); 4,01 (3H-s, $OCH_3$); 3,11 (3H-s, N-$CH_3$)

Beispiel 34

7-Chlor-4-hydroxy-2-methyl-N-(4-methyl-2-pyridyl)-2H-/1/benzothieno/2,3-e/-1,2-thiazin-3-carboxamid-1,1-dioxid

Hergestellt analog Beispiel 1 aus 7-Chlor-4-hydroxy-2-methyl-2H-[1]benzothieno[2,3-e]-1,2-thiazin-3-carbonsäuremethylester-1,1-dioxid und 2-Amino-4-methyl-pyridin in einer Ausbeute von 50 % der Theorie.

Schmelzpunkt: 232-233°C (Zersetzung) nach dem Umkristallisieren aus 1,2-Dichlorethan.

$C_{18}H_{14}ClN_3O_4S_2$ (435,91)

| | | | | | | | | | |
|---|---|---|---|---|---|---|---|---|---|
| Ber.: | C | 49,60 | H | 3,24 | N | 9,64 | S | 14,71 | Cl 8,13 |
| Gef.: | | 49,60 | | 3,35 | | 9,72 | | 14,68 | 8,67 |

IR (KBr): OH, NH breit, assoziiert, C=O 1650, $SO_2$ 1150, 1340/cm

UV (Ethanol): $\lambda_{max}$ 270 ($\varepsilon$ = 0,30), 300-330 ($\varepsilon$ = 0,30), 395 nm ($\varepsilon$ = 0,4); auf Zusatz von KOH: $\lambda_{max}$ 298 ($\varepsilon$ = 0,41), 400 nm ($\varepsilon$ = 0,4).

1H-NMR ($d_6$-DMSO): $\delta$ 14,70 (1H-s, austauschbares H); 8,54 (1H-d, J = 2 Hz, ar. H in 6-Stellung); 8,32 (1H-d, J = 6,6 Hz, ar. H in 6'-Stellung); 8,18 (1H-d, J = 9 Hz, ar. H in 9-Stellung); 7,9-7,6 (2H-m; ar. H in 8- und 3'-Stellung); 7,37 (1H-d von d, J = 6,6 und 1,5 Hz, ar. H in 5'-Stellung); 1 austauschbares H bei ca. 3,5 bis 5,0; 3,05 (3H-s, $N-CH_3$); 2,58 (3H-s; $\rangle C-CH_3$)

Beispiel 35

7-Chlor-4-hydroxy-2-methyl-N-(6-methyl-2-pyridyl)-2H-[1]benzothieno[2,3-e]-1,2-thiazin-3-carboxamid-1,1-dioxid

Herstellung analog Beispiel 1 aus 7-Chlor-4-hydroxy-2-methyl-2H-[1]benzothieno[2,3-e]-1,2-thiazin-3-carbonsäuremethylester-1,1-dioxid und 2-Amino-6-methyl-pyridin in einer Ausbeute von 50 % der Theorie.

Schmelzpunkt: 245-246°C (Zersetzung) nach dem Umkristallisieren aus Dimethylformamid/Methanol (4:1).

IR (KBr): assoziiertes N-H bzw. OH 3600 bis 2300, C = 0 1640, Amid-II 1560, $-SO_2-N-$ 1140, 1330/cm

UV (Ethanol): $\lambda_{max}$ (neutral) 255-275 (E = 0,14); 375-380 (E = 0,22) nm;
$\lambda_{max}$ (alkalisch) 300 (E = 0,38); 404 (E = 0,35 nm) Schulter bei 245 nm (E= 0,45)

(Konzentration: 60 µg/ml; Schichtdicke: 0,2 cm)

$^1$H-NMR ($CDCl_3$ + 10 % $CF_3CO_2{}^2H$; 80 MHz): $\delta$ 13,0 - 12,3 (2H, breit, austauschbare Protonen); 8,55-8,0 (4H-m; ar. H); 7,8-7,4 (2H-m; ar. H); 3,08 (3H-s; $N-CH_3$); 2,83 (3H-s; $C-CH_3$).

$C_{18}H_{14}ClN_3O_4S_2$ (435,91)

| | | | | | | | | | | |
|---|---|---|---|---|---|---|---|---|---|---|
| Ber.: | C | 49,60 | H | 3,24 | N | 9,64 | S | 14,71 | Cl | 8,13 |
| Gef.: | | 49,20 | | 3,39 | | 9,69 | | 14,78 | | 8,14 |

Beispiel 36

7-Chlor-4-hydroxy-2-methyl-N-(2-thiazolyl)-2H-/1/benzothieno-/2,3-e/-1,2-thiazin-3-carboxamid-1,1-dioxid

Hergestellt analog Beispiel 1 aus 7-Chlor-4-hydroxy-2-methyl-2H-/1/benzothieno/2,3-e/-1,2-thiazin-3-carbonsäuremethylester-1,1-dioxid und 2-Amino-thiazol in einer Ausbeute von 56 % der Theorie.

Schmelzpunkt: 259-260°C (Zersetzung) nach dem Umkristallisieren aus Dimethylformamid.

$C_{15}H_{10}N_3O_4S_3$ (427,91)

| | | | | | | | | | | |
|---|---|---|---|---|---|---|---|---|---|---|
| Ber.: | C | 42,10 | H | 2,36 | N | 9,82 | S | 22,48 | Cl | 8,28 |
| Gef.: | | 42,35 | | 2,48 | | 9,86 | | 22,35 | | 8,38 |

Beispiel 37

7-Chlor-4-hydroxy-2-methyl-N-(4-methyl-2-thiazolyl)-2H-/1/-benzothieno/2,3-e/-1,2-thiazin-3-carboxamid-1,1-dioxid

Hergestellt analog Beispiel 1 aus 7-Chlor-4-hydroxy-2-methyl-2H-/1/benzothieno/2,3-e/-1,2-thiazin-3-carbonsäuremethylester-1,1-dioxid und 2-Amino-4-methyl-thiazol in einer Ausbeute von 36 % der Theorie.

Schmelzpunkt: 233-234°C (Zersetzung) nach dem Umkristallisieren aus 1,2-Dichlorethan.

$C_{16}H_{12}ClN_3O_4S_3$ (441,94)

| | | | | | | | | | | |
|---|---|---|---|---|---|---|---|---|---|---|
| Ber.: | C | 43,48 | H | 2,74 | N | 9,51 | S | 21,77 | Cl | 8,02 |
| Gef.: | | 43,60 | | 2,83 | | 9,45 | | 21,50 | | 8,10 |

## Beispiel 38

7-Chlor-4-hydroxy-2-methyl-N-(5-methyl-2-thiazolyl)-2H-/1/-benzothieno/2,3-e/-1,2-thiazin-3-carboxamid-1,1-dioxid

Hergestellt analog Beispiel 1 aus 7-Chlor-4-hydroxy-2-methyl-2H-/1/benzothieno/2,3-e/-1,2-thiazin-3-carbonsäuremethylester-1,1-dioxid und 2-Amino-5-methyl-thiazol in einer Ausbeute von 42 % der Theorie.

Schmelzpunkt: 261 - 262°C (Zersetzung) nach dem Umkristallisieren aus Dimethylformamid.

$C_{16}H_{12}ClN_3O_4S_3$ (441,94)

| | | | | | | | | | | |
|---|---|---|---|---|---|---|---|---|---|---|
| Ber.: | C | 43,48 | H | 2,74 | N | 9,51 | S | 21,77 | Cl | 8,02 |
| Gef.: | | 43,60 | | 2,78 | | 9,63 | | 21,40 | | 8,35 |

## Beispiel 39

7-Chlor-4-hydroxy-2-methyl-N-(pyrazinyl)-2H-/1/benzothieno-/2,3-e/-1,2-thiazin-3-carboxamid-1,1-dioxid

Hergestellt analog Beispiel 1 aus 7-Chlor-4-hydroxy-2-methyl-2H-/1/benzothieno/2,3-e/-1,2-thiazin-3-carbonsäuremethylester-1,1-dioxid und Amino-pyrazin in einer Ausbeute von 70 % der Theorie.

Schmelzpunkt: 247-248°C (Zersetzung) nach dem Umkristallisieren aus Dimethylformamid.

$C_{16}H_{11}ClN_4O_4S_2$ (422,87)

| | | | | | | | | | | |
|---|---|---|---|---|---|---|---|---|---|---|
| Ber.: | C | 45,45 | H | 2,62 | N | 13,25 | S | 15,16 | Cl | 8,38 |
| Gef.: | | 45,20 | | 2,66 | | 12,95 | | 15,33 | | 8,40 |

Beispiel I

Tabletten mit 50 mg 4-Hydroxy-2-methyl-N-(2-pyridyl)-2H-/1/-benzothieno/2,3-e/-1,2-thiazin-3-carboxamid-1,1-dioxid

Zusammensetzung:

1 Tablette enthält

| | |
|---|---|
| Wirksubstanz | 50,0 mg |
| Maisstärke | 97,0 mg |
| Polyvinylpyrrolidon | 175,0 mg |
| Magnesiumstearat | 3,0 mg |
| | 325,0 mg |

Herstellungsverfahren:

Die Mischung der Wirksubstanz mit Maisstärke wird mit einer 14%igen Lösung des Polyvinylpyrrolidons in Wasser durch Sieb 1,5 mm granuliert, bei 45°C getrocknet und nochmals durch obiges Sieb gerieben. Das so erhaltene Granulat wird mit Magnesiumstearat gemischt und zu Tabletten verpreßt.

Tablettengewicht: 325 mg

Stempel: 10 mm, flach

Beispiel II

Dragées mit 50 mg 4-Hydroxy-2-methyl-N-(2-pyridyl)-2H-/1/-benzothieno/2,3-e/-1,2-thiazin-3-carboxamid-1,1-dioxid

Zusammensetzung:

1 Dragéekern enthält

| | |
|---|---|
| Wirksubstanz | 50,0 mg |
| Maisstärke | 245,0 mg |
| Gelatine | 8,0 mg |
| Talk | 18,0 mg |
| Magnesiumstearat | 4,0 mg |
| | 325,0 mg |

Herstellungsverfahren:

Die Mischung der Wirksubstanz mit Maisstärke wird mit einer 10%igen wäßrigen Gelatine-Lösung durch Sieb 1,5 mm granuliert, bei 45°C getrocknet und nochmals durch obiges Sieb gerieben. Das so erhaltene Granulat wird mit Talk und Magnesiumstearat gemischt und zu Drageekernen verpreßt.
Kerngewicht: 325 mg
Stempel: 10 mm, gewölbt
Die Drageekerne werden nach bekannten Verfahren mit einer Hülle überzogen, die im wesentlichen aus Zucker und Talkum besteht. Die fertigen Dragees werden mit Hilfe von Bienenwachs poliert.
Drageegewicht: 605 mg

Beispiel III

Gelatine-Kapseln mit 50 mg 4-Hydroxy-2-methyl-N-(4-methyl-2-pyridyl)-2H-[1]benzothieno[2,3-e]-1,2-thiazin-3-carboxamid-1,1-dioxid

Zusammensetzung:
1 Gelatine-Kapsel enthält

| | |
|---|---|
| Wirksubstanz | 50,0 mg |
| Maisstärke | 365,0 mg |
| Aerosil | 6,0 mg |
| Magnesiumstearat | 4,0 mg |
| | 425,0 mg |

Herstellungsverfahren:

Die Substanzen werden intensiv gemischt und in Gelatine-Kapseln Größe 1 abgefüllt.
Kapselinhalt: 425 mg

Beispiel IV

Suppositorien mit 50 mg 4-Hydroxy-2-methyl-N-(4-methyl-2-pyridyl)-2H-/1/benzothieno/2,3-e/-1,2-thiazin-3-carboxamid-1,1-dioxid

Zusammensetzung:

1 Zäpfchen enthält

| | |
|---|---|
| Wirksubstanz | 50,0 mg |
| Suppositorienmasse (z.B. Witepsol W 45) | 1 725,0 mg |
| | 1 775,0 mg |

Herstellungsverfahren:

Die feinpulverisierte Wirksubstanz wird mit Hilfe eines Eintauchhomogenisators in die geschmolzene und auf 40°C abgekühlte Zäpfchenmasse eingerührt. Die Masse wird bei 38°C in leicht vorgekühlte Formen gegossen.
Zäpfchengewicht: 1,775 g.

**P a t e n t a n s p r ü c h e :**

1. Neue 4-Hydroxy-2H/1/benzothieno/2,3-e/-1,2-thiazin-3-carboxamid-1,1-dioxide der allgemeinen Formel

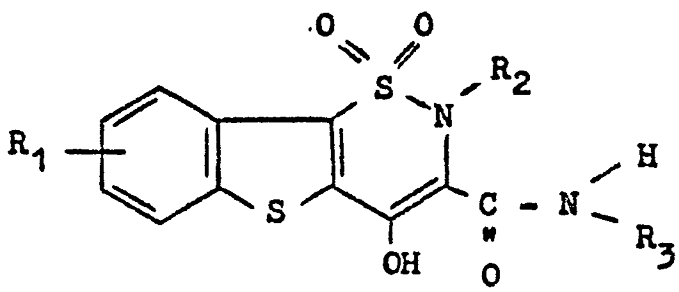

,(I)

in der

$R_1$ ein Wasserstoff- oder Halogenatom oder eine Alkylgruppe mit 1 bis 3 Kohlenstoffatomen,

$R_2$ ein Wasserstoffatom oder eine Alkylgruppe mit 1 bis 3 Kohlenstoffatomen und

$R_3$ einen aromatischen Kohlenwasserstoffrest mit 6 bis 10 Kohlenstoffatomen oder einen 1 oder 2 Stickstoffatome und/oder ein Sauerstoff- oder Schwefelatom enthaltenden heteroaromatischen Rest mit 2 bis 9 Kohlenstoffatomen, wobei die oben genannten aromatischen Reste durch 1 oder 2 Alkylreste mit 1 bis 6 Kohlenstoffatomen, durch ein Halogenatom, durch eine Hydroxy-, Trifluormethyl- oder Halogenphenylgruppe oder durch eine Alkoxygruppe mit 1 bis 3 Kohlenstoffatomen substituiert sein können, und deren physiologisch verträgliche Salze mit anorganischen oder organischen Basen.

2. Neue Verbindungen der allgemeinen Formel I gemäß Anspruch 1, in der $R_1$ und $R_2$ wie im Anspruch 1 definiert sind und $R_3$ eine Phenyl-, Naphthyl-, Pyridyl-, Pyrimidyl-, Pyrazinyl-, Pyridazinyl-, Oxazolyl-, Isoxazolyl-, Thiazolyl-, Isothiazolyl-, Benzothiazolyl-, 4,5,6,7-Tetrahydrobenzothiazolyl-, Cyclopentathiazolyl- oder 1,3,4-Thiadiazolylgruppe, die durch 1 oder 2 Alkylreste mit 1 bis 6 Kohlenstoffatomen, durch ein Halogenatom, durch eine Hydroxy-, Trifluormethyl- oder Halogenphenylgruppe oder durch eine Alkoxygruppe mit 1-3 Kohlenstoffatomen substituiert sein können, bedeutet, und deren physiologisch verträgliche Salze mit anorganischen oder organischen Basen.

3. Neue Verbindungen der allgemeinen Formel I gemäß Anspruch 1, in der
$R_1$ ein Wasserstoff-, Chloratom oder die Methylgruppe,
$R_2$ die Methylgruppe und
$R_3$ eine gegebenenfalls durch ein Chloratom substituierte Phenylgruppe, eine gegebenenfalls durch ein Chloratom, eine Hydroxy- oder Methylgruppe substituierte Pyridylgruppe, einen gegebenenfalls durch ein oder zwei Alkylgruppen mit 1 bis 4 Kohlenstoffatomen, ein Chloratom, eine Trifluormethyl- oder Chlorphenylgruppe substituierten Thiazolylrest, die Benzothiazolyl-, 5,6-Dimethylbenzothiazolyl-, 4,5,6,7-Tetrahydro-benzothiazolyl-, 5,6-Dihydro-4H-cyclopentathiazolyl-, 5-Methyl-isoxazolyl-, Pyrimidinyl- oder Pyrazinylgruppe bedeuten, und deren physiologisch verträgliche Salze mit anorganischen oder organischen Basen.

4. Neue Verbindungen der allgemeinen Formel I gemäß Anspruch 1, in der
   $R_1$ und $R_2$ wie im Anspruch 3 definiert sind und $R_1$ in 7-Position steht und
   $R_3$ die Thiazolyl-(2)-, Pyridyl-(2)-, 6-Methyl-pyridyl-(2)-, 4-Methyl-pyridyl-(2)-, 5-Chlor-thiazolyl-(2)-, 4-Methyl-thiazolyl-(2)- oder 4,5-Dimethyl-thiazolyl-(2)-gruppe darstellt, und deren physiologisch verträgliche Salze mit anorganischen oder organischen Basen.

5. 4-Hydroxy-2-methyl-N-(2-pyridyl)-2H-/1/-benzothieno/2,3-e/-1,2-thiazin-3-carboxamid-1,1-dioxid und dessen Salze mit anorganischen oder organischen Basen.

6. 4-Hydroxy-2-methyl-N-(4-methyl-2-pyridyl)-2H-/1/benzothieno/2,3-e/-1,2-thiazin-3-carboxamid-1,1-dioxid und dessen Salze mit anorganischen oder organischen Basen.

7. 7-Chlor-4-hydroxy-2-methyl-N-(6-methyl-2-pyridyl)-2H-/1/ benzothieno/2,3-e/-1,2-thiazin-3-carboxamid-1,1-dioxid und dessen Salze mit anorganischen oder organischen Basen.

8. 2,7-Dimethyl-4-hydroxy-N-(4-methyl-2-pyridyl)-2H-/1/benzothieno/2,3-e/1,2-thiazin-3-carboxamid-1,1-dioxid und dessen Salze mit anorganischen oder organischen Basen.

9. Arzneimittel, enthaltend eine Verbindung gemäß den Ansprüchen 1 bis 7 neben einem oder mehreren interten Trägerstoffen oder Verdünnungsmitteln.

10. Verwendung einer Verbindung gemäß den Ansprüchen 1 bis 7 zur Bekämpfung von entzündlichen Zuständen und/oder zur Prophylaxe arterieller Thromboembolien und arterieller Verschlußkrankheiten.

11. Verfahren zur Herstellung von neuen 4-Hydroxy-2H/1/benzothieno/2,3-e/-1,2-thiazin-3-carboxamid-1,1-dioxiden der allgemeinen Formel

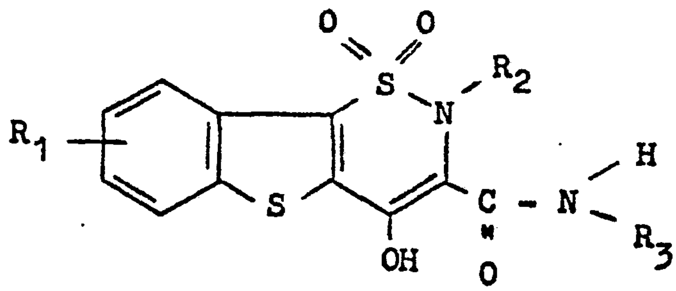

,(I)

in der

$R_1$ ein Wasserstoff- oder Halogenatom oder eine Alkylgruppe mit 1 bis 3 Kohlenstoffatomen,

$R_2$ ein Wasserstoffatom oder eine Alkylgruppe mit 1 bis 3 Kohlenstoffatomen und

$R_3$ einen aromatischen Kohlenwasserstoffrest mit 6 bis 10 Kohlenstoffatomen oder einen 1 oder 2 Stickstoffatome und/oder ein Sauerstoff- oder Schwefelatom enthaltenden heteroaromatischen Rest mit 2 bis 9 Kohlenstoffatomen, wobei die oben genannten aromatischen Reste durch 1 oder 2 Alkylreste mit 1 bis 6 Kohlenstoffatomen, durch ein Halogenatom, durch eine Hydroxy-, Trifluormethyl- oder Halogenphenylgruppe oder durch eine Alkoxygruppe mit 1-3 Kohlenstoffatomen substituiert sein können, bedeuten und von deren physiologisch verträglichen Salzen mit anorganischen oder organischen Basen, dadurch gekennzeichnet, daß ein 4-Hydroxy-2H/1/benzothieno/2,3-e/-1,2-thiazin-3-carbonsäure-Derivat der allgemeinen Formel

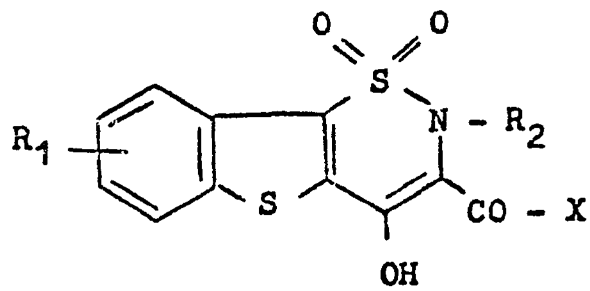

,(II)

in der

$R_1$ und $R_2$ wie eingangs definiert sind und

X eine nucleophil austauschbare Gruppe wie eine Alkoxygruppe mit 1 bis 6 Kohlenstoffatomen oder eine Aralkoxygruppe mit 7-10 Kohlenstoffatomen bedeutet, mit einem Amin der allgemeinen Formel

$$NH_2 - R_3$$ ,(III)

in der

$R_3$ wie eingangs definiert ist, umgesetzt wird und gewünschtenfalls eine so erhaltene Verbindung der allgemeinen Formel I in ihre physiologisch verträglichen Salze mit anorganischen oder organischen Basen übergeführt wird.

12. Verfahren gemäß Anspruch 11, dadurch gekennzeichnet, daß die Umsetzung in einem Lösungsmittel durchgeführt wird.

13. Verfahren gemäß Anspruch 11, dadurch gekennzeichnet, daß die Umsetzung bei Temperaturen zwischen 60 und 200°C, vorzugsweise jedoch bei der Siedetemperatur des Reaktionsgemisches, durchgeführt wird.

0009142

14. Verfahren gemäß den Ansprüchen 11, 12 und 13, dadurch gekennzeichnet, daß der bei der Umsetzung gebildete Alkohol HX aus dem Reaktionsgemisch durch azeotrope Destillation oder durch einen mit Molekularsieb beschickten Soxhlet-Extraktor entfernt wird.

0009142

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 79 103 150.3

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl.3) |
|---|---|---|---|
| | DE - A1 - 2 537 070 (F. HOFFMANN-LA ROCHE)<br>* Ansprüche 1, 28, 29 *<br>-- | 1,9,11 | C 07 D 513/04<br>A 61 K 31/54//<br>(C 07 D 513/04, 333/00, 279/00) |
| P | EP - A1 - 0 001 113 (F. HOFFMANN-LA ROCHE)<br>* Ansprüche 1, 19, 20 *<br>-- | 1,9,11 | |
| P | Chemical Abstracts Band 90, Nr. 3, 15. Januar 1979<br>Columbus, Ohio, USA<br>O. HROMATKA et al. "Thienothiazine derivatives",<br>Seite 659, Spalte 1, Abstract Nr. 23080y,<br>* Formel 1 *<br>& SU - A - 623 520 (F. HOFFMANN-LA ROCHE), 5. September 1978<br>-- | 1 | RECHERCHIERTE SACHGEBIETE (Int. Cl.3)<br>A 61 K 31/54<br>C 07 D 513/04 |
| A | DE - A1 - 2 452 996 (K. THOMAE)<br>--<br>../.. | | |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9, 11-14
Unvollständig recherchierte Patentansprüche: --
Nicht recherchierte Patentansprüche: 10
Grund für die Beschränkung der Recherche:

Verfahren zur therapeutischen Behandlung des menschlichen oder tierischen Körpers

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Berlin | 13-12-1979 | FROELICH |

EPA Form 1505.1 06.78

Europäisches Patentamt

**EUROPÄISCHER TEILRECHERCHENBERICHT**

Nummer der Anmeldung
EP 79 103 150.3
- Seite 2 -

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| A | DE - A1 - 2 539 112 (K. THOMAE)<br>---- | | |
| | | | RECHERCHIERTE SACHGEBIETE (Int.Cl.3) |